# EUROPEAN PATENT APPLICATION

(11) **EP 4 234 690 A1**
(43) Date of publication of application: **30.08.2023**
(21) Application number: 20958065.3
(22) Date of filing: 21.10.2020
(51) Int. Cl.: C12N 9/12, C12Q 1/68

(54) **RECOMBINANT KOD POLYMERASE**

(71) Applicant: BGI Shenzhen, Shenzhen, Guangdong 518083 (CN)
(72) Inventor: ZHAI, Lili, Shenzhen, Guangdong 518083 (CN); XIE, Qingqing, Shenzhen, Guangdong 518083 (CN); CAO, Ruyin, Shenzhen, Guangdong 518083 (CN); WANG, Zi, Shenzhen, Guangdong 518083 (CN); ZHENG, Yue, Shenzhen, Guangdong 518083 (CN); DONG, Yuliang, Shenzhen, Guangdong 518083 (CN); ZHANG, Wenwei, Shenzhen, Guangdong 518083 (CN); XU, Xun, Shenzhen, Guangdong 518083 (CN)
(74) Representative: Schwarz & Partner Patentanwälte GmbH
(86) International application number: PCT/CN2020/122380
(87) International publication number: WO 2022/082482

(57) **Abstract**

Provided is a recombinant KOD polymerase. A KOD polymerase mutant is a protein as follows: the protein is a protein having DNA polymerase activity that is obtained by modifying amino acid residues in at least one of the following 48 positions in the amino acid sequence of KOD DNA polymerase GH78: 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729, without changing other amino acid sequences; and compared with KOD DNA polymerase GH78, with regard to catalysis, the recombinant DNA polymerase exhibits a faster reaction rate, better catalytic efficiency, better affinity and other advantages, thereby improving the reaction rate of DNA polymerase in sequencing and increasing the reaction read length.

## Description

### FIELD

The present disclosure relates to the field of biological technology, and specifically relates to a recombinant KOD polymerase.

### BACKGROUND

DNA polymerase has a function of replicating DNA quickly and accurately, which plays an important role in passing genetic information precisely and maintaining the stability of genetic material in living organism. There are five types of DNA polymerases in *E. coli* and are divided into A, B, C and other types according to the sequence homology of these DNA polymerases. The complete folding structure of DNA polymerase is similar with a human hand, which is divided into three parts: palm, thumb and finger. Although the structures of the fingers and palm domains in different types of DNA polymerases vary greatly, the palm domains for catalysis differ slightly. The amino acid sequences of the active sites of DNA polymerase are mainly conserved, but these conserved active sites are also relatively easy to be changed, and unnatural specific dNTPs can be incorporated to keep the activity of DNA polymerase from decreasing significantly (refer to Pat. No. PCT/CN2018/103764).

KOD DNA polymerase belonging to B-family DNA polymerase is a heat-resistant DNA polymerase replicating DNA quickly and accurately. The KOD DNA polymerase has a wide range of applications, and one of the most important applications is in genome sequencing, such as SBS sequencing, which use nucleotides modified in the 3' hydroxyl group in the pentose to block the addition of other nucleotides. The use of nucleotides with 3' blocking groups allows the incorporation of nucleotides into the polynucleotide chain in a controlled manner. After the addition of each nucleotide, the presence of the 3' blocking group prevents other nucleotides from being incorporated into the chain. After removing the blocking group, the natural 3' hydroxyl group is released freely for adding the next nucleotide. However, there are many technical defects with current SBS sequencing, such as short read length of sequencing and slow reaction rate.

### SUMMARY

The present disclosure aims at improvements for accelerating biochemical reaction steps in sequencing, shortening reaction time and so on, and modifies the KOD DNA polymerase more deeply based on the previous research, in which original functional sites of B-family DNA polymerase related are protected, thereby ensuring the polymerase still performing its originally basic functions. The present disclosure in embodiments performed a dynamic simulation in terms of the palm, thumb and finger domains of the KOD DNA polymerase, and obtained mutant sites that could be used for experimental screening, according to statistical inference. Further, experimental program was designed with semi-rational design, enzyme variant library construction and high-throughput screening to improve the catalytic, physical and chemical properties of polymerase for developing DNA polymerases suitable for DNB (DNA nano ball) sequencing attached to the chip surface. Finally, the recombinant DNA polymerases were isolated and purified.

Solutions as follows are provided in embodiments of the present disclosure for obtaining a DNA polymerase for catalyzing with higher reaction rate or adding nucleotides with unnatural modifications of 3' blocking groups during SBS sequencing.

A first object of the present disclosure is to provide a mutant of a KOD DNA polymerase.

According to embodiments of the present disclosure, the mutant of the KOD DNA polymerase is a protein having DNA polymerase activity, wherein
the protein has an amino acid sequence with modification on one or more of amino acid residue selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on a KOD DNA polymerase GH78, and with other amino acid residues unchanged,
the KOD DNA polymerase GH78 is set forth in SEQ ID NO: 1,
optionally, the protein is further added with a first tag sequence at the terminal of the protein.

According to some embodiments, the protein may have the amino acid sequence with the modification on at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from the group consisting of 48 positions at 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged.

The modification is substitution of an amino acid.

According to some embodiments, the substitutions of amino acid in individual proteins are as follows:
T at position 267 is substituted with D;
F at position 326 is substituted with L;
S at position 347 is substituted with D or V;
T at position 349 is substituted with G;
V at position 353 is substituted with D;
K at position 375 is substituted with I, A or E;
A at position 378 is substituted with E, Q or K;
R at position 379 is substituted with K, Q, E, H or Y;
R at position 380 is substituted with E, D, S, G, N or Y;
E at position 385 is substituted with F or R;
S at position 451 is substituted with N, T, G, H, M, L, P, R, A, Q or K;
L at position 452 is substituted with V;
L at position 453 is substituted with A, T, V or I;
G at position 454 is substituted with Q;
L at position 457 is substituted with M;
Q at position 461 is substituted with A or S;
K at position 465 is substituted with Q;
T at position 470 is substituted with S;
L at position 474 is substituted with K, V, A or T;
K at position 477 is substituted with E;
L at position 478 is mutated to I, V, Y, M, C, N, H or G;
L at position 479 is mutated to M;
D at position 480 is mutated to S, R, T, N, G, F, H or E;
R at position 482 is mutated to K;
R at position 484 is mutated to C, D, I, V, K or G;
E at position 485 is mutated to I, C or F;
I at position 486 is mutated to K or R;
Y at position 493 is mutated to F, H or L;
Y at position 496 is mutated to T or M;
Y at position 497 is mutated to L, F, M, I or W;
T at position 514 was mutated to A;
A at position 574 is mutated to L;
K at position 584 was mutated to T;
T at position 605 is mutated to W or V;
I at position 610 is mutated to L or M;
L at position 630 is mutated to F;
a glutamic acid at position 665 is mutated to R, S, K or C;
an asparagine at position 666 is mutated to A, N, Q, T or K;
T at position 667 is mutated to R, N or S;
K at position 674 is mutated to Q, M, I, E or R;
K at position 676 is mutated to S, L, R, H, V, I, M, A or T;
M at position 680 is mutated to I, L, T or A;
V at position 682 is mutated to M;
V at position 698 is mutated to I;
S at position 707 is mutated to G, T or K;
D at position 718 is mutated to Q;
T at position 723 is mutated to V, C, G, I or F;
A at position 729 is mutated to T.

According to some embodiments, the protein may be any one of the following KOD DNA polymerase mutants, which has DNA polymerase activity and has an amino acid sequence with modification on one or more of amino acid residue selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the KOD DNA polymerase mutant has an identifier and mutated position(s) thereof shown as follows:
GH101, an amino acid sequence with D at position 480 of SEQ ID NO: 1 mutated to N and with other amino acid residues unchanged;
GH102, an amino acid sequence with K at position 676 of SEQ ID NO: 1 mutated to H and with other amino acid residues unchanged;
GH103, an amino acid sequence with E at position 485 of SEQ ID NO: 1 mutated to I and with other amino acid residues unchanged;
GH104, an amino acid sequence with Y at position 497 of SEQ ID NO: 1 mutated to L and with other amino acid residues unchanged;
GH105, an amino acid sequence with S at position 451 of SEQ ID NO: 1 mutated to Q and with other amino acid residues unchanged;
GH106, an amino acid sequence with R at position 482 of SEQ ID NO: 1 mutated to K and with other amino acid residues unchanged;
GH107, an amino acid sequence with L at position 453 of SEQ ID NO: 1 mutated to V and with other amino acid residues unchanged;
GH108, an amino acid sequence with I at position 474 of SEQ ID NO: 1 mutated to T and with other amino acid residues unchanged;
GH109, an amino acid sequence with T at position 267 of SEQ ID NO: 1 mutated to D and with other amino acid residues unchanged;
GH110, an amino acid sequence with T at position 723 of SEQ ID NO: 1 mutated to V and with other amino acid residues unchanged;
GH111, an amino acid sequence with R at position 379 of SEQ ID NO: 1 mutated to Y and with other amino acid residues unchanged;
GH112, an amino acid sequence with L at position 453 of SEQ ID NO: 1 mutated to I and with other amino acid residues unchanged;
GH113, an amino acid sequence with I at position 474 of SEQ ID NO: 1 mutated to K and with other amino acid residues unchanged;
GH114, an amino acid sequence with V at position 353 of SEQ ID NO: 1 mutated to D and with other amino acid residues unchanged;
GH115, an amino acid sequence with S at position 347 of SEQ ID NO: 1 mutated to D and with other amino acid residues unchanged;
GH116, an amino acid sequence with K at position 465 of SEQ ID NO: 1 mutated to Q and with other amino acid residues unchanged;
GH117, an amino acid sequence with R at position 380 of SEQ ID NO: 1 mutated to D and with other amino acid residues unchanged;
GH118, an amino acid sequence with Q at position 461 of SEQ ID NO: 1 mutated to A and with other amino acid residues unchanged;
GH119, an amino acid sequence with I at position 610 of SEQ ID NO: 1 mutated to L and with other amino acid residues unchanged;
GH120, an amino acid sequence with T at position 723 of SEQ ID NO: 1 mutated to G and with other amino acid residues unchanged;
GH121, an amino acid sequence with Y at position 496 of SEQ ID NO: 1 mutated to M and with other amino acid residues unchanged;
GH122, an amino acid sequence with K at position 375 of SEQ ID NO: 1 mutated to A and with other amino acid residues unchanged;
GH123, an amino acid sequence with Y at position 497 of SEQ ID NO: 1 mutated to I and with other amino acid residues unchanged;
GH124, an amino acid sequence with I at position 474 of SEQ ID NO: 1 mutated to A and with other amino acid residues unchanged;
GH125, an amino acid sequence with S at position 707 of SEQ ID NO: 1 mutated to G and with other amino acid residues unchanged;
GH126, an amino acid sequence with R at position 379 of SEQ ID NO: 1 mutated to E and with other amino acid residues unchanged;
GH127, an amino acid sequence with T at position 723 of SEQ ID NO: 1 mutated to I and with other amino acid residues unchanged;
GH128, an amino acid sequence with Q at position 461 of SEQ ID NO: 1 mutated to S and with other amino acid residues unchanged;
GH129, an amino acid sequence with D at position 718 of SEQ ID NO: 1 mutated to Q and with other amino acid residues unchanged;
GH131, an amino acid sequence with L at position 452 of SEQ ID NO: 1 mutated to V and with other amino acid residues unchanged;
GH132, an amino acid sequence with R at position 380 of SEQ ID NO: 1 mutated to Y and with other amino acid residues unchanged;
GH133, an amino acid sequence with L at position 478 of SEQ ID NO: 1 mutated to V and with other amino acid residues unchanged;
GH134, an amino acid sequence with L at position 478 of SEQ ID NO: 1 mutated to Y and with other amino acid residues unchanged;
GH135, an amino acid sequence with E at position 385 of SEQ ID NO: 1 mutated to R and with other amino acid residues unchanged;
GH136, an amino acid sequence with I at position 474 of SEQ ID NO: 1 mutated to V and with other amino acid residues unchanged;
GH137, an amino acid sequence with L at position 479 of SEQ ID NO: 1 mutated to M and with other amino acid residues unchanged;
GH138, an amino acid sequence with M at position 680 of SEQ ID NO: 1 mutated to L and with other amino acid residues unchanged;
GH139, an amino acid sequence with L at position 478 of SEQ ID NO: 1 mutated to I and with other amino acid residues unchanged;
GH140, an amino acid sequence with T at position 723 of SEQ ID NO: 1 mutated to C and with other amino acid residues unchanged;
GH141, an amino acid sequence with L at position 478 of SEQ ID NO: 1 mutated to M and with other amino acid residues unchanged;
GH142, an amino acid sequence with A at position 729 of SEQ ID NO: 1 mutated to T and with other amino acid residues unchanged;
GH143, an amino acid sequence with I at position 610 of SEQ ID NO: 1 mutated to M and with other amino acid residues unchanged;
GH144, an amino acid sequence with V at position 353 of SEQ ID NO: 1 mutated to D and with other amino acid residues unchanged;
GH145, an amino acid sequence with K at position 676 of SEQ ID NO: 1 mutated to S and with other amino acid residues unchanged;
GH146, an amino acid sequence with F at position 326 of SEQ ID NO: 1 mutated to L and with other amino acid residues unchanged;
GH147, an amino acid sequence with A at position 574 of SEQ ID NO: 1 mutated to L and with other amino acid residues unchanged;
GH148, an amino acid sequence with L at position 630 of SEQ ID NO: 1 mutated to F and with other amino acid residues unchanged; and
GH149, an amino acid sequence with T at position 349 of SEQ ID NO: 1 mutated to G and with other amino acid residues unchanged.

According to some embodiments, the protein may have DNA polymerase activity and have an amino acid sequence with modification on at least two amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
KH222, an amino acid sequence with E mutated to I at position 485 and S mutated to L at position 451 of SEQ ID NO: 1 and with other amino acid residues unchanged;
KH223, an amino acid sequence with E mutated to I at position 485 and S mutated to T at position 451 of SEQ ID NO: 1 and with other amino acid residues unchanged;
KH224, an amino acid sequence with E mutated to I at position 485 and D mutated to C at position 484 of SEQ ID NO: 1 and with other amino acid residues unchanged;
KH225, an amino acid sequence with E mutated to I at position 485 and D mutated to T at position 480 of SEQ ID NO: 1 and with other amino acid residues unchanged;
KH226, an amino acid sequence with E mutated to I at position 485 and S mutated to G at position 451 of SEQ ID NO: 1 and with other amino acid residues unchanged;
KH227, an amino acid sequence with E mutated to I at position 485 and L mutated to A at position 453 of SEQ ID NO: 1 and with other amino acid residues unchanged;
KH228, an amino acid sequence with E mutated to C at position 485 and R mutated to V at position 484 of SEQ ID NO: 1 and with other amino acid residues unchanged;
KH229, an amino acid sequence with E mutated to C at position 485 and S mutated to N at position 451 of SEQ ID NO: 1 and with other amino acid residues unchanged;
KH230, an amino acid sequence with E mutated to C at position 485 and D mutated to N at position 480 of SEQ ID NO: 1 and with other amino acid residues unchanged;
KH232, an amino acid sequence with E mutated to C at position 485 and R mutated to I at position 484 of SEQ ID NO: 1 and with other amino acid residues unchanged;
KH233, an amino acid sequence with K mutated to S at position 676 and M mutated to I at position 680 of SEQ ID NO: 1 and with other amino acid residues unchanged;
KH234, an amino acid sequence with E mutated to C at position 485 and I mutated to R at position 486 of SEQ ID NO: 1 and with other amino acid residues unchanged;
KH235, an amino acid sequence with E mutated to I at position 485 and D mutated to S at position 480 of SEQ ID NO: 1 and with other amino acid residues unchanged;
KH236, an amino acid sequence with Y mutated to F at position 493 and Y mutated to L at position 497 of SEQ ID NO: 1 and with other amino acid residues unchanged;
KH237, an amino acid sequence with K mutated to L at position 676 and M mutated to I at position 680 of SEQ ID NO: 1 and with other amino acid residues unchanged;
KH238, an amino acid sequence with E mutated to I at position 485 and D mutated to Q at position 480 of SEQ ID NO: 1 and with other amino acid residues unchanged;
KH239, an amino acid sequence with E mutated to I at position 485 and D mutated to M at position 480 of SEQ ID NO: 1 and with other amino acid residues unchanged;
KH240, an amino acid sequence with E mutated to I at position 485 and S mutated to K at position 451 of SEQ ID NO: 1 and with other amino acid residues unchanged;
KH241, an amino acid sequence with E mutated to I at position 485 and D mutated to R at position 480 of SEQ ID NO: 1 and with other amino acid residues unchanged;
KH242, an amino acid sequence with E mutated to I at position 485 and L mutated to N at position 453 of SEQ ID NO: 1 and with other amino acid residues unchanged;
KH243, an amino acid sequence with K mutated to R at position 676 and M mutated to I at position 680 of SEQ ID NO: 1 and with other amino acid residues unchanged;
KH244, an amino acid sequence with E mutated to I at position 485 and S mutated to M at position 451 of SEQ ID NO: 1 and with other amino acid residues unchanged;
KH245, an amino acid sequence with E mutated to I at position 485 and K mutated to E at position 477 of SEQ ID NO: 1 and with other amino acid residues unchanged;
KH246, an amino acid sequence with E mutated to I at position 485 and D mutated to W at position 480 of SEQ ID NO: 1 and with other amino acid residues unchanged;
KH247, an amino acid sequence with E mutated to I at position 485 and D mutated to K at position 480 of SEQ ID NO: 1 and with other amino acid residues unchanged;
KH248, an amino acid sequence with E mutated to I at position 485 and S mutated to R at position 451 of SEQ ID NO: 1 and with other amino acid residues unchanged;
KH249, an amino acid sequence with K mutated to L at position 676 and M mutated to L at position 680 of SEQ ID NO: 1 and with other amino acid residues unchanged;
KH250, an amino acid sequence with E mutated to I at position 485 and Q mutated to R at position 665 of SEQ ID NO: 1 and with other amino acid residues unchanged;
KH251, an amino acid sequence with E mutated to I at position 485 and Q mutated to S at position 665 of SEQ ID NO: 1 and with other amino acid residues unchanged;
KH252, an amino acid sequence with E mutated to I at position 485 and D mutated to E at position 480 of SEQ ID NO: 1 and with other amino acid residues unchanged;
KH253, an amino acid sequence with K mutated to H at position 676 and M mutated to I at position 680 of SEQ ID NO: 1 and with other amino acid residues unchanged;
KH254, an amino acid sequence with E mutated to I at position 485 and Q mutated to A at position 461 of SEQ ID NO: 1 and with other amino acid residues unchanged;
KH255, an amino acid sequence with E mutated to I at position 485 and S mutated to A at position 451 of SEQ ID NO: 1 and with other amino acid residues unchanged;
KH256, an amino acid sequence with E mutated to I at position 485 and S mutated to Q at position 451 of SEQ ID NO: 1 and with other amino acid residues unchanged;
KH257, an amino acid sequence with E mutated to I at position 485 and I mutated to Q at position 666 of SEQ ID NO: 1 and with other amino acid residues unchanged;
KH258, an amino acid sequence with E mutated to I at position 485 and I mutated to T at position 666 of SEQ ID NO: 1 and with other amino acid residues unchanged;
KH259, an amino acid sequence with E mutated to I at position 485 and T mutated to W at position 605 of SEQ ID NO: 1 and with other amino acid residues unchanged;
KH260, an amino acid sequence with E mutated to I at position 485 and R mutated to Y at position 379 of SEQ ID NO: 1 and with other amino acid residues unchanged;
KH261, an amino acid sequence with E mutated to I at position 485 and L mutated to T at position 453 of SEQ ID NO: 1 and with other amino acid residues unchanged;
KH262, an amino acid sequence with E mutated to I at position 485 and D mutated to C at position 480 of SEQ ID NO: 1 and with other amino acid residues unchanged;
KH263, an amino acid sequence with E mutated to I at position 485 and T mutated to V at position 605 of SEQ ID NO: 1 and with other amino acid residues unchanged;
KH264, an amino acid sequence with E mutated to I at position 485 and D mutated to N at position 480 of SEQ ID NO: 1 and with other amino acid residues unchanged;
KH266, an amino acid sequence with E mutated to I at position 485 and D mutated to G at position 480 of SEQ ID NO: 1 and with other amino acid residues unchanged;
KH267, an amino acid sequence with K mutated to Q at position 465 and D mutated to Q at position 718 of SEQ ID NO: 1 and with other amino acid residues unchanged;
KH268, an amino acid sequence with E mutated to I at position 485 and R mutated to H at position 379 of SEQ ID NO: 1 and with other amino acid residues unchanged;
KH269, an amino acid sequence with E mutated to I at position 485 and K mutated to Q at position 465 of SEQ ID NO: 1 and with other amino acid residues unchanged;
KH270, an amino acid sequence with E mutated to I at position 485 and D mutated to P at position 480 of SEQ ID NO: 1 and with other amino acid residues unchanged;
KH271, an amino acid sequence with E mutated to I at position 485 and D mutated to F at position 480 of SEQ ID NO: 1 and with other amino acid residues unchanged;
KH272, an amino acid sequence with K mutated to V at position 676 and M mutated to L at position 680 of SEQ ID NO: 1 and with other amino acid residues unchanged;
KH273, an amino acid sequence with E mutated to I at position 485 and L mutated to V at position 453 of SEQ ID NO: 1 and with other amino acid residues unchanged;
KH274, an amino acid sequence with E mutated to I at position 485 and D mutated to H at position 480 of SEQ ID NO: 1 and with other amino acid residues unchanged;
KH275, an amino acid sequence with E mutated to I at position 485 and S mutated to P at position 451 of SEQ ID NO: 1 and with other amino acid residues unchanged;
KH276, an amino acid sequence with E mutated to I at position 485 and Q mutated to K at position 665 of SEQ ID NO: 1 and with other amino acid residues unchanged;
KH277, an amino acid sequence with D mutated to S at position 480 and S mutated to Q at position 451 of SEQ ID NO: 1 and with other amino acid residues unchanged;
KH278, an amino acid sequence with I mutated to T at position 474 and L mutated to C at position 478 of SEQ ID NO: 1 and with other amino acid residues unchanged;
KH279, an amino acid sequence with K mutated to H at position 676 and M mutated to T at position 680 of SEQ ID NO: 1 and with other amino acid residues unchanged;
KH280, an amino acid sequence with E mutated to I at position 485 and S mutated to V at position 347 of SEQ ID NO: 1 and with other amino acid residues unchanged;
KH281, an amino acid sequence with D mutated to N at position 480 and L mutated to V at position 453 of SEQ ID NO: 1 and with other amino acid residues unchanged;
KH282, an amino acid sequence with E mutated to I at position 485 and I mutated to A at position 666 of SEQ ID NO: 1 and with other amino acid residues unchanged;
KH283, an amino acid sequence with K mutated to V at position 676 and M mutated to I at position 680 of SEQ ID NO: 1 and with other amino acid residues unchanged;
KH284, an amino acid sequence with K mutated to I at position 676 and M mutated to L at position 680 of SEQ ID NO: 1 and with other amino acid residues unchanged;
KH285, an amino acid sequence with K mutated to M at position 676 and M mutated to A at position 680 of SEQ ID NO: 1 and with other amino acid residues unchanged;
KH286, an amino acid sequence with E mutated to I at position 485 and T mutated to S at position 470 of SEQ ID NO: 1 and with other amino acid residues unchanged;
KH287, an amino acid sequence with K mutated to L at position 676 and M mutated to A at position 680 of SEQ ID NO: 1 and with other amino acid residues unchanged;
KH288, an amino acid sequence with R mutated to K at position 379 and R mutated to E at position 380 of SEQ ID NO: 1 and with other amino acid residues unchanged;
KH201, an amino acid sequence with D mutated to N at position 480 and M mutated to I at position 680 of SEQ ID NO: 1 and with other amino acid residues unchanged;
KH202, an amino acid sequence with I mutated to T at position 474 and L mutated to N at position 478 of SEQ ID NO: 1 and with other amino acid residues unchanged;
KH203, an amino acid sequence with I mutated to T at position 474 and L mutated to H at position 478 of SEQ ID NO: 1 and with other amino acid residues unchanged;
KH204, an amino acid sequence with E mutated to I at position 485 and R mutated to K at position 484 of SEQ ID NO: 1 and with other amino acid residues unchanged;
KH205, an amino acid sequence with Y mutated to H at position 493 and Y mutated to F at position 497 of SEQ ID NO: 1 and with other amino acid residues unchanged;
KH206, an amino acid sequence with Y mutated to F at position 493 and Y mutated to M at position 497 of SEQ ID NO: 1 and with other amino acid residues unchanged;
KH207, an amino acid sequence with T mutated to A at position 514 and K mutated to T at position 584 of SEQ ID NO: 1 and with other amino acid residues unchanged;
KH208, an amino acid sequence with I mutated to T at position 474 and L mutated to G at position 478 of SEQ ID NO: 1 and with other amino acid residues unchanged;
KH209, an amino acid sequence with E mutated to I at position 485 and Q mutated to C at position 665 of SEQ ID NO: 1 and with other amino acid residues unchanged;
KH210, an amino acid sequence with K mutated to Q at position 465 and K mutated to T at position 584 of SEQ ID NO: 1 and with other amino acid residues unchanged;
KH211, an amino acid sequence with K mutated to M at position 676 and M mutated to L at position 680 of SEQ ID NO: 1 and with other amino acid residues unchanged;
KH212, an amino acid sequence with Y mutated to H at position 493 and Y mutated to L at position 497 of SEQ ID NO: 1 and with other amino acid residues unchanged;
KH213, an amino acid sequence with L mutated to M at position 457 and Q mutated to A at position 461 of SEQ ID NO: 1 and with other amino acid residues unchanged;
KH214, an amino acid sequence with K mutated to S at position 676 and M mutated to A at position 680 of SEQ ID NO: 1 and with other amino acid residues unchanged;
KH125, an amino acid sequence with E mutated to F at position 485 and I mutated to K at position 486 of SEQ ID NO: 1 and with other amino acid residues unchanged;
KH126, an amino acid sequence with D mutated to N at position 480 and K mutated to T at position 584 of SEQ ID NO: 1 and with other amino acid residues unchanged;
KH217, an amino acid sequence with K mutated to E at position 375 and A mutated to K at position 378 of SEQ ID NO: 1 and with other amino acid residues unchanged;
KH218, an amino acid sequence with Y mutated to F at position 493 and Y mutated to I at position 497 of SEQ ID NO: 1 and with other amino acid residues unchanged;
KH219, an amino acid sequence with I mutated to K at position 474 and L mutated to H at position 478 of SEQ ID NO: 1 and with other amino acid residues unchanged;
KH220, an amino acid sequence with V mutated to D at position 353 and Y mutated to T at position 496 of SEQ ID NO: 1 and with other amino acid residues unchanged;
KH221, an amino acid sequence with R mutated to Q at position 379 and R mutated to D at position 380 of SEQ ID NO: 1 and with other amino acid residues unchanged;
QH349, an amino acid sequence with K mutated to A at position 676 and M mutated to L at position 680 of SEQ ID NO: 1 and with other amino acid residues unchanged;
QH350, an amino acid sequence with D mutated to N at position 480 and R mutated to K at position 482 of SEQ ID NO: 1 and with other amino acid residues unchanged;
QH351, an amino acid sequence with S mutated to Q at position 451 and L mutated to V at position 453 of SEQ ID NO: 1 and with other amino acid residues unchanged;
QH352, an amino acid sequence with I mutated to K at position 474 and L mutated to Y at position 478 of SEQ ID NO: 1 and with other amino acid residues unchanged;
QH353, an amino acid sequence with D mutated to N at position 480 and T mutated to D at position 267 of SEQ ID NO: 1 and with other amino acid residues unchanged;
QH354, an amino acid sequence with K mutated to L at position 676 and M mutated to T at position 680 of SEQ ID NO: 1 and with other amino acid residues unchanged;
QH355, an amino acid sequence with E mutated to I at position 485 and G mutated to Q at position 454 of SEQ ID NO: 1 and with other amino acid residues unchanged;
QH356, an amino acid sequence with K mutated to V at position 676 and V mutated to M at position 682 of SEQ ID NO: 1 and with other amino acid residues unchanged;
QH357, an amino acid sequence with K mutated to Q at position 465 and S mutated to G at position 707 of SEQ ID NO: 1 and with other amino acid residues unchanged;
QH358, an amino acid sequence with K mutated to I at position 676 and M mutated to I at position 680 of SEQ ID NO: 1 and with other amino acid residues unchanged;
QH359, an amino acid sequence with I mutated to K at position 474 and L mutated to G at position 478 of SEQ ID NO: 1 and with other amino acid residues unchanged;
QH360, an amino acid sequence with R mutated to E at position 379 and R mutated to S at position 380 of SEQ ID NO: 1 and with other amino acid residues unchanged;
QH361, an amino acid sequence with K mutated to I at position 375 and A mutated to Q at position 378 of SEQ ID NO: 1 and with other amino acid residues unchanged;
QH362, an amino acid sequence with Y mutated to H at position 493 and Y mutated to M at position 497 of SEQ ID NO: 1 and with other amino acid residues unchanged;
QH363, an amino acid sequence with K mutated to A at position 676 and M mutated to I at position 680 of SEQ ID NO: 1 and with other amino acid residues unchanged;
QH329, an amino acid sequence with E mutated to I at position 485 and R mutated to G at position 484 of SEQ ID NO: 1 and with other amino acid residues unchanged;
QH364, an amino acid sequence with K mutated to I at position 676 and M mutated to T at position 680 of SEQ ID NO: 1 and with other amino acid residues unchanged;
QH365, an amino acid sequence with I mutated to K at position 474 and L mutated to V at position 478 of SEQ ID NO: 1 and with other amino acid residues unchanged;
QH370, an amino acid sequence with E mutated to I at position 485 and I mutated to Q at position 666 of SEQ ID NO: 1 and with other amino acid residues unchanged; and
QH371, an amino acid sequence with E mutated to I at position 485 and I mutated to R at position 666 of SEQ ID NO: 1 and with other amino acid residues unchanged.

According to some embodiments, the protein may have DNA polymerase activity and have an amino acid sequence with modification on at least three amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH303, an amino acid sequence with K mutated to L at position 676, M mutated to T at position 680, and V mutated to I at position 689 of SEQ ID NO: 1 and with other amino acid residues unchanged;
QH304, an amino acid sequence with L mutated to M at position 457, Q mutated to A at position 461, and T mutated to D at position 267 of SEQ ID NO: 1 and with other amino acid residues unchanged;
QH305, an amino acid sequence with K mutated to Q at position 465, K mutated to T at position 584, and E mutated to I at position 485 of SEQ ID NO: 1 and with other amino acid residues unchanged;
QH306, an amino acid sequence with K mutated to Q at position 465, D mutated to Q at position 718, and E mutated to I at position 485 of SEQ ID NO: 1 and with other amino acid residues unchanged;
QH307, an amino acid sequence with L mutated to M at position 457, Q mutated to A at position 461, and E mutated to I at position 485 of SEQ ID NO: 1 and with other amino acid residues unchanged;
QH308, an amino acid sequence with K mutated to S at position 676, M mutated to I at position 680, and E mutated to I at position 485 of SEQ ID NO: 1 and with other amino acid residues unchanged;
QH309, an amino acid sequence with Y mutated to F at position 493, Y mutated to L at position 497, and E mutated to I at position 485 of SEQ ID NO: 1 and with other amino acid residues unchanged;
QH310, an amino acid sequence with D mutated to N at position 480, S mutated to Q at position 451, and D mutated to Q at position 718 of SEQ ID NO: 1 and with other amino acid residues unchanged;
QH311, an amino acid sequence with D mutated to N at position 480, K mutated to M at position 674, and M mutated to I at position 680 of SEQ ID NO: 1 and with other amino acid residues unchanged;
QH312, an amino acid sequence with D mutated to N at position 480, S mutated to Q at position 451, and E mutated to I at position 485 of SEQ ID NO: 1 and with other amino acid residues unchanged;
QH313, an amino acid sequence with D mutated to N at position 480, K mutated to Q at position 465, and D mutated to Q at position 718 of SEQ ID NO: 1 and with other amino acid residues unchanged; and
QH314, an amino acid sequence with D mutated to N at position 480, S mutated to Q at position 451, and Q mutated to A at position 461 of SEQ ID NO: 1 and with other amino acid residues unchanged.

According to some embodiments, the protein may have DNA polymerase activity and have an amino acid sequence with modification on at least four amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH315, an amino acid sequence with K mutated to Q at position 465, D mutated to Q at position 718, L mutated to M at position 457, and Q mutated to A at position 461 of SEQ ID NO: 1 and with other amino acid residues unchanged; and
QH316, an amino acid sequence with K mutated to Q at position 465, D mutated to Q at position 718, D mutated to N at position 480, and E mutated to I at position 485 of SEQ ID NO: 1 and with other amino acid residues unchanged.

According to some embodiments, the protein may have DNA polymerase activity and have an amino acid sequence with modification on at least three amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH317, an amino acid sequence with D mutated to N at position 480, S mutated to Q at position 451, and R mutated to Y at position 379 of SEQ ID NO: 1 and with other amino acid residues unchanged;
QH318, an amino acid sequence with L mutated to M at position 457, Q mutated to A at position 461, and K mutated to Q at position 465 of SEQ ID NO: 1 and with other amino acid residues unchanged;
QH319, an amino acid sequence with D mutated to N at position 480, K mutated to Q at position 674, and M mutated to I at position 680 of SEQ ID NO: 1 and with other amino acid residues unchanged;
QH320, an amino acid sequence with D mutated to N at position 480, K mutated to R at position 674, and M mutated to I at position 680 of SEQ ID NO: 1 and with other amino acid residues unchanged;
QH321, an amino acid sequence with D mutated to N at position 480, S mutated to Q at position 451, and K mutated to Q at position 465 of SEQ ID NO: 1 and with other amino acid residues unchanged;
QH322, an amino acid sequence with M mutated to I at position 680, K mutated to H at position 676, and E mutated to I at position 485 of SEQ ID NO: 1 and with other amino acid residues unchanged;
QH323, an amino acid sequence with K mutated to Q at position 465, D mutated to Q at position 718, and T mutated to D at position 267 of SEQ ID NO: 1 and with other amino acid residues unchanged;
QH324, an amino acid sequence with S mutated to Q at position 451, L mutated to V at position 452, and L mutated to V at position 453 of SEQ ID NO: 1 and with other amino acid residues unchanged;
QH325, an amino acid sequence with S mutated to Q at position 451, K mutated to M at position 674, and K mutated to T at position 676 of SEQ ID NO: 1 and with other amino acid residues unchanged;
QH326, an amino acid sequence with K mutated to Q at position 465, D mutated to Q at position 718, and R mutated to Y at position 379 of SEQ ID NO: 1 and with other amino acid residues unchanged;
QH326, an amino acid sequence with K mutated to Q at position 465, D mutated to Q at position 718, and L mutated to V at position 453 of SEQ ID NO: 1 and with other amino acid residues unchanged;
QH328, an amino acid sequence with D mutated to N at position 480, K mutated to I at position 674, and M mutated to I at position 680 of SEQ ID NO: 1 and with other amino acid residues unchanged;
QH331, an amino acid sequence with K mutated to Q at position 465, K mutated to T at position 584, and L mutated to V at position 453 of SEQ ID NO: 1 and with other amino acid residues unchanged;
QH332, an amino acid sequence with K mutated to Q at position 465, D mutated to Q at position 718, and S mutated to G at position 707 of SEQ ID NO: 1 and with other amino acid residues unchanged;
QH333, an amino acid sequence with K mutated to Q at position 465, K mutated to T at position 584, and R mutated to Y at position 379 of SEQ ID NO: 1 and with other amino acid residues unchanged;
QH334, an amino acid sequence with S mutated to Q at position 451, R mutated to K at position 482, and K mutated to T at position 676 of SEQ ID NO: 1 and with other amino acid residues unchanged;
QH335, an amino acid sequence with K mutated to Q at position 465, K mutated to T at position 584, and T mutated to D at position 267 of SEQ ID NO: 1 and with other amino acid residues unchanged; and
QH336, an amino acid sequence with E mutated to I at position 485, K mutated to L at position 676, and M mutated to L at position 680 of SEQ ID NO: 1 and with other amino acid residues unchanged.

According to some embodiments, the protein may have DNA polymerase activity and have an amino acid sequence with modification on at least four amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH338, an amino acid sequence with D mutated to N at position 480, T mutated to R at position 667, K mutated to Q at position 674, and M mutated to I at position 680 of SEQ ID NO: 1 and with other amino acid residues unchanged;
QH339, an amino acid sequence with D mutated to N at position 480, T mutated to N at position 667, K mutated to M at position 674, and M mutated to I at position 680 of SEQ ID NO: 1 and with other amino acid residues unchanged;
QH340, an amino acid sequence with D mutated to N at position 480, T mutated to S at position 667, K mutated to M at position 674, and M mutated to I at position 680 of SEQ ID NO: 1 and with other amino acid residues unchanged;
QH341, an amino acid sequence with S mutated to Q at position 451, D mutated to N at position 480, K mutated to Q at position 674, and M mutated to I at position 680 of SEQ ID NO: 1 and with other amino acid residues unchanged;
QH342, an amino acid sequence with E mutated to I at position 485, K mutated to L at position 676, M mutated to T at position 680, and V mutated to I at position 698 of SEQ ID NO: 1 and with other amino acid residues unchanged;
QH343, an amino acid sequence with D mutated to N at position 480, T mutated to N at position 667, K mutated to Q at position 674, and M mutated to I at position 680 of SEQ ID NO: 1 and with other amino acid residues unchanged;
QH344, an amino acid sequence with K mutated to S at position 676, M mutated to I at position 680, Y mutated to F at position 493, and Y mutated to L at position 497 of SEQ ID NO: 1 and with other amino acid residues unchanged; and
QH345, an amino acid sequence with S mutated to Q at position 451, R mutated to K at position 482, K mutated to Q at position 674, and M mutated to L at position 680 of SEQ ID NO: 1 and with other amino acid residues unchanged.

According to some embodiments, the protein may have DNA polymerase activity and have an amino acid sequence with modification on at least five amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH346, an amino acid sequence with S mutated to Q at position 451, R mutated to K at position 482, K mutated to M at position 674, M mutated to L at position 680, and L mutated to M at position 479 of SEQ ID NO: 1 and with other amino acid residues unchanged; and
QH347, an amino acid sequence with S mutated to Q at position 451, L mutated to I at position 453, D mutated to N at position 480, K mutated to Q at position 674, and M mutated to I at position 680 of SEQ ID NO: 1 and with other amino acid residues unchanged.

According to some embodiments, the protein may have DNA polymerase activity and have an amino acid sequence with modification on at least six amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged. For example, the protein has an identifier and mutated position(s) thereof shown as follows:
QH348, an amino acid sequence with S mutated to Q at position 451, R mutated to K at position 482, K mutated to Q at position 674, M mutated to L at position 680, L mutated to V at position 452, and D mutated to N at position 480 of SEQ ID NO: 1 and with other amino acid residues unchanged.

A nucleic acid encoding the protein as described above also falls into the protection scope of the present disclosure.

The nucleic acid may be a DNA molecule which has a first nucleotide sequence encoding a mutant of a KOD DNA polymerase GH78 as set forth in SEQ ID NO: 1, wherein the first nucleotide sequence has amino-acid-codon substitution on a second nucleotide sequence encoding the KOD DNA polymerase GH78 at at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729,
the second nucleotide sequence is set forth in SEQ ID NO: 2,
optionally, the DNA molecule is further added with a second tag sequence at the terminal of the DNA molecule.

An expression cassette, a recombinant vector, a recombinant bacterium or a transgenic cell line containing the DNA molecule as described above also fall into the protection scope of the present disclosure.

Use of the protein as described above in one or more of the following i-v also falls into the protection scope of the present disclosure:
i. serving as a DNA polymerase;
ii. catalyzing a DNA replication and/or DNA amplification;
iii. performing a nucleic acid sequencing;
iv. preparing a product for catalyzing a DNA replication and/or DNA amplification; and
v. preparing a product for a nucleic acid sequencing.

Use of the DNA molecule or the expression cassette, recombinant vector, recombinant bacterium or transgenic cell line as described above in one or more of the following i-vi also falls into the protection scope of the present disclosure:
i. preparing a DNA polymerase;
ii. catalyzing a DNA replication and/or DNA amplification;
iii. performing a nucleic acid sequencing;
iv. preparing a product of a DNA polymerase;
v. preparing a product for catalyzing a DNA replication and/or DNA amplification; and
vi. preparing a product for a nucleic acid sequencing.

According to the use in some embodiments, the sequencing is SBS sequencing, and the nucleic acid is a DNA, RNA or whole genome.

According to the use in some embodiments, the product is a kit.

### DESCRIPTION OF THE DRAWINGS

Figure 1 is a diagram showing a structure of recombinant KOD DNA polymerase, in which "1" represents a singal peptide, "2" represents a His tag for purifying a fusion protein; and "3" represents a KOD DNA polymerase GH78.
Figure 2 shows the measurement of the purity of wild-type KOD DNA polymerase fusion protein.

### DESCRIPTION OF EXEMPLARY EMBODIMENTS

The experimental methods used in the following examples are conventional methods unless otherwise specified.

The materials and reagents used in the following examples are commercially available unless otherwise specified.

### Example 1. Preparation of KOD DNA Polymerase Mutant

In Examples of the present disclosure, the expression vectors containing KOD DNA polymerase or mutants thereof were constructed with DNA 2.0 Electra^{™} Cloning Reagents Kit, and His tag was used for Ni column affinity purification.

### 1. Preparation of KOD DNA Polymerase GH78

The amino acid sequence of the KOD DNA polymerase is set forth as SEQ ID NO: 1 in the sequence listing and the gene sequence encoding it is set forth as SEQ ID NO: 2 in the sequence listing.

### 1.1 Construction of GH78 Expression Vector pD441-WT

The recombinant expression vector pD441-78 was obtained by recombining the gene encoding the KOD DNA polymerase fused with His tag to a pD441-pelB vector (DNA2.0, pD441-pelB) with operation steps according to the instruction of Electra^{™} Cloning Reagents Kit (DNA2.0, EKT-02). The gene encoding the KOD DNA polymerase fused with the His tag was expressed by guiding by the signal peptide in the pD441-pelB vector.

The nucleotide sequence of the gene encoding the KOD DNA polymerase fused with His tag was a sequence linked with codons of 6×His tag at the 3' end of SEQ ID NO: 2.

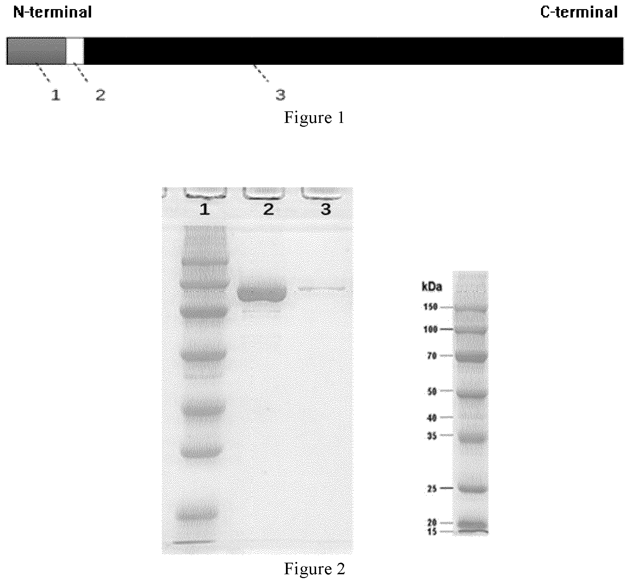

The amino acid sequence of the KOD DNA polymerase GH78 fusion protein was obtained by connecting 6× His tag with the N-terminal of the amino acid of SEQ ID NO: 1. As shown in Figure 1, "1" represents the signal peptide pelB; "2" represents the His tag for purifying the fusion protein; and "3" represents the KOD DNA polymerase GH78 (i.e. SEQ ID NO: 1).

### 1.2 Construction of Recombinant Bacteria

The recombinant expression vector pD441-78 was transformed into *E. coli* BL21 competent cells (purchased from TransGen Biotech Co., Ltd.), and coated on a plate containing 50 µg/ml of kanamycin to select positive colonies. 3-5 positive colonies were selected and then identified with PCR, using primers of Cloning-F (i.e. SEQ ID NO: 3 in the sequence listing) and Cloning-R (i.e. SEQ ID NO: 4 in the sequence listing). A fragment with a size of 2,800 bp, which is basically consistent with a predicted theoretical value by aligning the sequencing results, was obtained and determined as a positive clone, and named as BL21/pD441-78.
Cloning-F: 5'GGTTTTTTTATGGGGGGAGTTTAGG 3' (SEQ ID NO: 3)
Cloning-R: 5'CATCTCATCTGTAACATCATTGGCA 3' (SEQ ID NO: 4)

### 1.3 Expression and Purification of the KOD DNA polymerase GH78 fusion protein

A single colony of BL21/pD441-78 was selected and cultured overnight at 37° C in 50 ml LB liquid medium containing 50 µg/ml of kanamycin with 220 rpm/min. On the next day, the cultured bacteria was diluted at 1:100 and transferred to 1 L of LB liquid medium containing 50 µg/ml of kanamycin, then cultured in a shaker at 37° C and 220 rpm/min to OD₆₀₀ of 0.5-0.8. Then, 0.5 mM of IPTG in a final concentration was added to induce the expression of the fusion protein at 25° C. overnight, and induced BL21/pD441-78 bacterial solution was obtained. The IPTG was not added to the bacterial solution of the blank control group.

The above BL21/pD441-78 bacterial solution was centrifuged at 8000 rpm/min for 10 minutes for discarding the supernatant, and pelleted bacterial cells were collected and resuspended in a buffer at pH 7.0 and prepared with 50 mM KPO₄, 500 mM NaCl, 10 mM imidazole, 5% Glycerol (percentage by volume and water for making up), and PMSF at final concentration of 0.5 mM was added additionally. Then the bacteria cells was broken by a ultrasonic disruptor, with a working time of 40 min, power of 200 W, ultrasonic disruptor of 1 s and stop of 2 s and alarm temperature of 15° C. After sonication, the disrupted bacteria cells were placed in water bath at 80° C for 20 min, during which it should be noticed to regularly mix the solution so that the disrupted bacteria cells were heated evenly. Then the solution was centrifuged at 12,000 rpm/min at 4° C for 30 min, and the supernatant was filtered with a 0.22 µm filter membrane, and the filtrate was collected to obtain crude cell extract.

The crude cell extract was loaded at an appropriate flow rate for Ni column affinity chromatography with a prepacked column of affinity chromatography (HisTrap FF, 5 ml, 17-5255-01, GE healthcare), and the loaded column was equilibrated with 5 CV of buffer 1, and then eluted with 5 CV of 3% buffer 2 (containing 50 m MKPO₄, 1 M NaCl, 5% Glycerol, pH 7.0), followed by eluting with 5 CV of 50% buffer 2. The eluate of the Ni column affinity chromatography corresponding to the peak value equal to or higher than 100 mAU was collected.

The eluate corresponding to the peak value equal to or higher than 100 mAU was loaded at a certain flow rate for ion exchange chromatography with a prepacked column of ion exchange chromatography (HiTrap Q HP, 5 ml, GE healthcare), and the loaded column was equilibrated with 5 CV of buffer 2, and linearly eluted with 0% buffer 2 - 60% buffer 2. The eluate of the ion exchange chromatography corresponding to the peak value equal to or higher than 100 mAU was collected.

The eluate of the ion exchange chromatography corresponding to the peak value equal to or higher than 100 mAU was subjected to gel chromatography (HiTrap SP HP, 5ml, GE healthcare), in which the column was washed with 3 CV of 20% ethanol, followed by 3 CV with water, and equilibrated with 3 CV of 100% buffer 3 (containing 20 mM Tris, 200 mM KCl, 0.2 mM EDTA and 10% Glycerol, pH 7.4). After that, the sample was loaded, and the column was eluted with 1.5 CV of buffer 3, and the collected eluate was purified KOD DNA polymerase fusion protein.

The purified KOD DNA polymerase fusion protein was subjected to SDS-PAGE (5% stacking gel and 12% separation gel), where the protein was mixed with loading buffer (5×), treated at 95° C for 5 min, and then loaded.

The results are shown in Figure 2, in which lane 1 shows a protein Marker (PageRuler Prestained Protein Ladder, 26616, Thermo Scientific), lane 2 shows 10 µl of 1 mg/ml purified KOD DNA polymerase GH78 fusion protein, and lane 3 shows 10 µl of 0.05 mg/ml purified KOD DNA polymerase GH78 fusion protein that were diluted 20 times. It can be seen from the results of protein electrophoresis that the protein size in both lane 2 and lane 3 are about 91.5 KDa, which is consistent with the molecular weight reported in the literature.

The purity of the protein after the gel electrophoresis was analyzed with Quantity one software, and the purity of the KOD DNA polymerase GH78 fusion protein reached to 95% or above, after purification.

The target protein with the size of about 91.5 KDa was not observed in uninduced BL21/pD441-78 bacterial solution.

In the control group, a blank vector of pD441-pelB was transformed into *E. coli* BL21 to obtain BL21/pD441-pelB. The above method was used to express and purify the protein of the control group, but the target protein of about 91.5 KDa was not obtained as well.

### 2. Preparation of KOD DNA Polymerase Mutant Fusion Protein

The KOD DNA polymerase mutant is a protein with substitution of amino acid on at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729, based on the amino acid sequence of GH 78 set forth as SEQ ID NO: 1. The protein is a DNA polymerase mutant with a single site mutation if there is only 1 amino-acid substitution; the protein is a DNA polymerase mutant with a two-site recombinant mutation if there are 2 amino-acid substitutions; the protein is a DNA polymerase mutant with a three-site recombinant mutation if there are 3 amino-acid substitutions, and so on.

The gene encoding the KOD DNA polymerase mutant has a nucleotide sequence with corresponding amino-acid-codon substitution on the nucleotide sequence of the KOD DNA polymerase GH78 set forth in SEQ ID NO: 2 at at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729, based on the KOD DNA polymerase set forth in SEQ ID NO: 1.

The mutant position and type of the KOD DNA polymerase mutant with single-site mutation are set forth in Table 1 for example.

**Table 1 Mutant position and type of the KOD DNA polymerase mutant with single-site mutation**

| Indentifer of KOD DNA polymerase mutant | Mutated position (corresponding to the position in SEQ ID NO: 1) | Original amino acid in the KOD DNA polymerase GH78 | Mutated amino acid in the KOD DNA polymerase mutant |
|---|---|---|---|
| GH101 | 480 | D | N |
| GH102 | 676 | K | H |
| GH103 | 485 | E | I |
| GH104 | 497 | Y | L |
| GH105 | 451 | S | Q |
| GH106 | 482 | R | K |
| GH107 | 453 | L | V |
| GH108 | 474 | I | T |
| GH109 | 267 | T | D |
| GH110 | 723 | T | V |
| GH111 | 379 | R | Y |
| GH112 | 453 | L | I |
| GH113 | 474 | I | K |
| GH114 | 353 | V | D |
| GH115 | 347 | S | D |
| GH116 | 465 | K | Q |
| GH117 | 380 | R | D |
| GH118 | 461 | Q | A |
| GH119 | 610 | I | L |
| GH120 | 723 | T | G |
| GH121 | 496 | Y | M |
| GH122 | 375 | K | A |
| GH123 | 497 | Y | I |
| GH124 | 474 | I | A |
| GH125 | 707 | S | G |
| GH126 | 379 | R | E |
| GH127 | 723 | T | I |
| GH128 | 461 | Q | S |
| GH129 | 718 | D | Q |
| GH131 | 452 | L | V |
| GH132 | 380 | R | Y |
| GH133 | 478 | L | V |
| GH134 | 478 | L | Y |
| GH135 | 385 | E | R |
| GH136 | 474 | I | V |
| GH137 | 479 | L | M |
| GH138 | 680 | M | L |
| GH139 | 478 | L | I |
| GH140 | 723 | T | C |
| GH141 | 478 | L | M |
| GH142 | 729 | A | T |
| GH143 | 610 | I | M |
| GH144 | 353 | V | D |
| GH145 | 676 | K | S |
| GH146 | 326 | F | L |
| GH147 | 574 | A | L |
| GH148 | 630 | L | F |
| GH149 | 349 | T | G |

For example, the mutant positions and types in Table 1 may be described as follows: for the KOD DNA polymerase mutant GH101, it is a protein with amino-acid substitution of aspartic acid (D) to asparagine (N) on 480th amino acid of the amino acid sequence of the KOD DNA polymerase GH78 (i.e. SEQ ID NO: 1).

For the KOD DNA polymerase mutant GH101, its coding gene has a nucleotide sequence with corresponding amino-acid-codon substitution on the nucleotide sequence of the KOD DNA polymerase GH78 set forth in SEQ ID NO: 2, in which the nucleotide encoding aspartic acid (D) is mutated to asparagine (N) at the 480th amino acid of the KOD DNA polymerase set forth in SEQ ID NO: 1.

Specific mutated positions and types of the DNA polymerase mutant with a two-site recombinant mutation based on the KOD DNA polymerase GH78 are as follows:
KH222, an amino acid sequence with E mutated to I at position 485 and S mutated to L at position 451 of SEQ ID NO: 1 and with other amino acid residues unchanged;
KH223, an amino acid sequence with E mutated to I at position 485 and S mutated to T at position 451 of SEQ ID NO: 1 and with other amino acid residues unchanged;
KH224, an amino acid sequence with E mutated to I at position 485 and D mutated to C at position 484 of SEQ ID NO: 1 and with other amino acid residues unchanged;
KH225, an amino acid sequence with E mutated to I at position 485 and D mutated to T at position 480 of SEQ ID NO: 1 and with other amino acid residues unchanged;
KH226, an amino acid sequence with E mutated to I at position 485 and S mutated to G at position 451 of SEQ ID NO: 1 and with other amino acid residues unchanged;
KH227, an amino acid sequence with E mutated to I at position 485 and L mutated to A at position 453 of SEQ ID NO: 1 and with other amino acid residues unchanged;
KH228, an amino acid sequence with E mutated to C at position 485 and R mutated to V at position 484 of SEQ ID NO: 1 and with other amino acid residues unchanged;
KH229, an amino acid sequence with E mutated to C at position 485 and S mutated to N at position 451 of SEQ ID NO: 1 and with other amino acid residues unchanged;
KH230, an amino acid sequence with E mutated to C at position 485 and D mutated to N at position 480 of SEQ ID NO: 1 and with other amino acid residues unchanged;
KH232, an amino acid sequence with E mutated to C at position 485 and R mutated to I at position 484 of SEQ ID NO: 1 and with other amino acid residues unchanged;
KH233, an amino acid sequence with K mutated to S at position 676 and M mutated to I at position 680 of SEQ ID NO: 1 and with other amino acid residues unchanged;
KH234, an amino acid sequence with E mutated to C at position 485 and I mutated to R at position 486 of SEQ ID NO: 1 and with other amino acid residues unchanged;
KH235, an amino acid sequence with E mutated to I at position 485 and D mutated to S at position 480 of SEQ ID NO: 1 and with other amino acid residues unchanged;
KH236, an amino acid sequence with Y mutated to F at position 493 and Y mutated to L at position 497 of SEQ ID NO: 1 and with other amino acid residues unchanged;
KH237, an amino acid sequence with K mutated to L at position 676 and M mutated to I at position 680 of SEQ ID NO: 1 and with other amino acid residues unchanged;
KH238, an amino acid sequence with E mutated to I at position 485 and D mutated to Q at position 480 of SEQ ID NO: 1 and with other amino acid residues unchanged;
KH239, an amino acid sequence with E mutated to I at position 485 and D mutated to M at position 480 of SEQ ID NO: 1 and with other amino acid residues unchanged;
KH240, an amino acid sequence with E mutated to I at position 485 and S mutated to K at position 451 of SEQ ID NO: 1 and with other amino acid residues unchanged;
KH241, an amino acid sequence with E mutated to I at position 485 and D mutated to R at position 480 of SEQ ID NO: 1 and with other amino acid residues unchanged;
KH242, an amino acid sequence with E mutated to I at position 485 and L mutated to N at position 453 of SEQ ID NO: 1 and with other amino acid residues unchanged;
KH243, an amino acid sequence with K mutated to R at position 676 and M mutated to I at position 680 of SEQ ID NO: 1 and with other amino acid residues unchanged;
KH244, an amino acid sequence with E mutated to I at position 485 and S mutated to M at position 451 of SEQ ID NO: 1 and with other amino acid residues unchanged;
KH245, an amino acid sequence with E mutated to I at position 485 and K mutated to E at position 477 of SEQ ID NO: 1 and with other amino acid residues unchanged;
KH246, an amino acid sequence with E mutated to I at position 485 and D mutated to W at position 480 of SEQ ID NO: 1 and with other amino acid residues unchanged;
KH247, an amino acid sequence with E mutated to I at position 485 and D mutated to K at position 480 of SEQ ID NO: 1 and with other amino acid residues unchanged;
KH248, an amino acid sequence with E mutated to I at position 485 and S mutated to R at position 451 of SEQ ID NO: 1 and with other amino acid residues unchanged;
KH249, an amino acid sequence with K mutated to L at position 676 and M mutated to L at position 680 of SEQ ID NO: 1 and with other amino acid residues unchanged;
KH250, an amino acid sequence with E mutated to I at position 485 and Q mutated to R at position 665 of SEQ ID NO: 1 and with other amino acid residues unchanged;
KH251, an amino acid sequence with E mutated to I at position 485 and Q mutated to S at position 665 of SEQ ID NO: 1 and with other amino acid residues unchanged;
KH252, an amino acid sequence with E mutated to I at position 485 and D mutated to E at position 480 of SEQ ID NO: 1 and with other amino acid residues unchanged;
KH253, an amino acid sequence with K mutated to H at position 676 and M mutated to I at position 680 of SEQ ID NO: 1 and with other amino acid residues unchanged;
KH254, an amino acid sequence with E mutated to I at position 485 and Q mutated to A at position 461 of SEQ ID NO: 1 and with other amino acid residues unchanged;
KH255, an amino acid sequence with E mutated to I at position 485 and S mutated to A at position 451 of SEQ ID NO: 1 and with other amino acid residues unchanged;
KH256, an amino acid sequence with E mutated to I at position 485 and S mutated to Q at position 451 of SEQ ID NO: 1 and with other amino acid residues unchanged;
KH257, an amino acid sequence with E mutated to I at position 485 and I mutated to Q at position 666 of SEQ ID NO: 1 and with other amino acid residues unchanged;
KH258, an amino acid sequence with E mutated to I at position 485 and I mutated to T at position 666 of SEQ ID NO: 1 and with other amino acid residues unchanged;
KH259, an amino acid sequence with E mutated to I at position 485 and T mutated to W at position 605 of SEQ ID NO: 1 and with other amino acid residues unchanged;
KH260, an amino acid sequence with E mutated to I at position 485 and R mutated to Y at position 379 of SEQ ID NO: 1 and with other amino acid residues unchanged;
KH261, an amino acid sequence with E mutated to I at position 485 and L mutated to T at position 453 of SEQ ID NO: 1 and with other amino acid residues unchanged;
KH262, an amino acid sequence with E mutated to I at position 485 and D mutated to C at position 480 of SEQ ID NO: 1 and with other amino acid residues unchanged;
KH263, an amino acid sequence with E mutated to I at position 485 and T mutated to V at position 605 of SEQ ID NO: 1 and with other amino acid residues unchanged;
KH264, an amino acid sequence with E mutated to I at position 485 and D mutated to N at position 480 of SEQ ID NO: 1 and with other amino acid residues unchanged;
KH266, an amino acid sequence with E mutated to I at position 485 and D mutated to G at position 480 of SEQ ID NO: 1 and with other amino acid residues unchanged;
KH267, an amino acid sequence with K mutated to Q at position 465 and D mutated to Q at position 718 of SEQ ID NO: 1 and with other amino acid residues unchanged;
KH268, an amino acid sequence with E mutated to I at position 485 and R mutated to H at position 379 of SEQ ID NO: 1 and with other amino acid residues unchanged;
KH269, an amino acid sequence with E mutated to I at position 485 and K mutated to Q at position 465 of SEQ ID NO: 1 and with other amino acid residues unchanged;
KH270, an amino acid sequence with E mutated to I at position 485 and D mutated to P at position 480 of SEQ ID NO: 1 and with other amino acid residues unchanged;
KH271, an amino acid sequence with E mutated to I at position 485 and D mutated to F at position 480 of SEQ ID NO: 1 and with other amino acid residues unchanged;
KH272, an amino acid sequence with K mutated to V at position 676 and M mutated to L at position 680 of SEQ ID NO: 1 and with other amino acid residues unchanged;
KH273, an amino acid sequence with E mutated to I at position 485 and L mutated to V at position 453 of SEQ ID NO: 1 and with other amino acid residues unchanged;
KH274, an amino acid sequence with E mutated to I at position 485 and D mutated to H at position 480 of SEQ ID NO: 1 and with other amino acid residues unchanged;
KH275, an amino acid sequence with E mutated to I at position 485 and S mutated to P at position 451 of SEQ ID NO: 1 and with other amino acid residues unchanged;
KH276, an amino acid sequence with E mutated to I at position 485 and Q mutated to K at position 665 of SEQ ID NO: 1 and with other amino acid residues unchanged;
KH277, an amino acid sequence with D mutated to S at position 480 and S mutated to Q at position 451 of SEQ ID NO: 1 and with other amino acid residues unchanged;
KH278, an amino acid sequence with I mutated to T at position 474 and L mutated to C at position 478 of SEQ ID NO: 1 and with other amino acid residues unchanged;
KH279, an amino acid sequence with K mutated to H at position 676 and M mutated to T at position 680 of SEQ ID NO: 1 and with other amino acid residues unchanged;
KH280, an amino acid sequence with E mutated to I at position 485 and S mutated to V at position 347 of SEQ ID NO: 1 and with other amino acid residues unchanged;
KH281, an amino acid sequence with D mutated to N at position 480 and L mutated to V at position 453 of SEQ ID NO: 1 and with other amino acid residues unchanged;
KH282, an amino acid sequence with E mutated to I at position 485 and I mutated to A at position 666 of SEQ ID NO: 1 and with other amino acid residues unchanged;
KH283, an amino acid sequence with K mutated to V at position 676 and M mutated to I at position 680 of SEQ ID NO: 1 and with other amino acid residues unchanged;
KH284, an amino acid sequence with K mutated to I at position 676 and M mutated to L at position 680 of SEQ ID NO: 1 and with other amino acid residues unchanged;
KH285, an amino acid sequence with K mutated to M at position 676 and M mutated to A at position 680 of SEQ ID NO: 1 and with other amino acid residues unchanged;
KH286, an amino acid sequence with E mutated to I at position 485 and T mutated to S at position 470 of SEQ ID NO: 1 and with other amino acid residues unchanged;
KH287, an amino acid sequence with K mutated to L at position 676 and M mutated to A at position 680 of SEQ ID NO: 1 and with other amino acid residues unchanged;
KH288, an amino acid sequence with R mutated to K at position 379 and R mutated to E at position 380 of SEQ ID NO: 1 and with other amino acid residues unchanged;
KH201, an amino acid sequence with D mutated to N at position 480 and M mutated to I at position 680 of SEQ ID NO: 1 and with other amino acid residues unchanged;
KH202, an amino acid sequence with I mutated to T at position 474 and L mutated to N at position 478 of SEQ ID NO: 1 and with other amino acid residues unchanged;
KH203, an amino acid sequence with I mutated to T at position 474 and L mutated to H at position 478 of SEQ ID NO: 1 and with other amino acid residues unchanged;
KH204, an amino acid sequence with E mutated to I at position 485 and R mutated to K at position 484 of SEQ ID NO: 1 and with other amino acid residues unchanged;
KH205, an amino acid sequence with Y mutated to H at position 493 and Y mutated to F at position 497 of SEQ ID NO: 1 and with other amino acid residues unchanged;
KH206, an amino acid sequence with Y mutated to F at position 493 and Y mutated to M at position 497 of SEQ ID NO: 1 and with other amino acid residues unchanged;
KH207, an amino acid sequence with T mutated to A at position 514 and K mutated to T at position 584 of SEQ ID NO: 1 and with other amino acid residues unchanged;
KH208, an amino acid sequence with I mutated to T at position 474 and L mutated to G at position 478 of SEQ ID NO: 1 and with other amino acid residues unchanged;
KH209, an amino acid sequence with E mutated to I at position 485 and Q mutated to C at position 665 of SEQ ID NO: 1 and with other amino acid residues unchanged;
KH210, an amino acid sequence with K mutated to Q at position 465 and K mutated to T at position 584 of SEQ ID NO: 1 and with other amino acid residues unchanged;
KH211, an amino acid sequence with K mutated to M at position 676 and M mutated to L at position 680 of SEQ ID NO: 1 and with other amino acid residues unchanged;
KH212, an amino acid sequence with Y mutated to H at position 493 and Y mutated to L at position 497 of SEQ ID NO: 1 and with other amino acid residues unchanged;
KH213, an amino acid sequence with L mutated to M at position 457 and Q mutated to A at position 461 of SEQ ID NO: 1 and with other amino acid residues unchanged;
KH214, an amino acid sequence with K mutated to S at position 676 and M mutated to A at position 680 of SEQ ID NO: 1 and with other amino acid residues unchanged;
KH125, an amino acid sequence with E mutated to F at position 485 and I mutated to K at position 486 of SEQ ID NO: 1 and with other amino acid residues unchanged;
KH126, an amino acid sequence with D mutated to N at position 480 and K mutated to T at position 584 of SEQ ID NO: 1 and with other amino acid residues unchanged;
KH217, an amino acid sequence with K mutated to E at position 375 and A mutated to K at position 378 of SEQ ID NO: 1 and with other amino acid residues unchanged;
KH218, an amino acid sequence with Y mutated to F at position 493 and Y mutated to I at position 497 of SEQ ID NO: 1 and with other amino acid residues unchanged;
KH219, an amino acid sequence with I mutated to K at position 474 and L mutated to H at position 478 of SEQ ID NO: 1 and with other amino acid residues unchanged;
KH220, an amino acid sequence with V mutated to D at position 353 and Y mutated to T at position 496 of SEQ ID NO: 1 and with other amino acid residues unchanged;
KH221, an amino acid sequence with R mutated to Q at position 379 and R mutated to D at position 380 of SEQ ID NO: 1 and with other amino acid residues unchanged;
QH349, an amino acid sequence with K mutated to A at position 676 and M mutated to L at position 680 of SEQ ID NO: 1 and with other amino acid residues unchanged;
QH350, an amino acid sequence with D mutated to N at position 480 and R mutated to K at position 482 of SEQ ID NO: 1 and with other amino acid residues unchanged;
QH351, an amino acid sequence with S mutated to Q at position 451 and L mutated to V at position 453 of SEQ ID NO: 1 and with other amino acid residues unchanged;
QH352, an amino acid sequence with I mutated to K at position 474 and L mutated to Y at position 478 of SEQ ID NO: 1 and with other amino acid residues unchanged;
QH353, an amino acid sequence with D mutated to N at position 480 and T mutated to D at position 267 of SEQ ID NO: 1 and with other amino acid residues unchanged;
QH354, an amino acid sequence with K mutated to L at position 676 and M mutated to T at position 680 of SEQ ID NO: 1 and with other amino acid residues unchanged;
QH355, an amino acid sequence with E mutated to I at position 485 and G mutated to Q at position 454 of SEQ ID NO: 1 and with other amino acid residues unchanged;
QH356, an amino acid sequence with K mutated to V at position 676 and V mutated to M at position 682 of SEQ ID NO: 1 and with other amino acid residues unchanged;
QH357, an amino acid sequence with K mutated to Q at position 465 and S mutated to G at position 707 of SEQ ID NO: 1 and with other amino acid residues unchanged;
QH358, an amino acid sequence with K mutated to I at position 676 and M mutated to I at position 680 of SEQ ID NO: 1 and with other amino acid residues unchanged;
QH359, an amino acid sequence with I mutated to K at position 474 and L mutated to G at position 478 of SEQ ID NO: 1 and with other amino acid residues unchanged;
QH360, an amino acid sequence with R mutated to E at position 379 and R mutated to S at position 380 of SEQ ID NO: 1 and with other amino acid residues unchanged;
QH361, an amino acid sequence with K mutated to I at position 375 and A mutated to Q at position 378 of SEQ ID NO: 1 and with other amino acid residues unchanged;
QH362, an amino acid sequence with Y mutated to H at position 493 and Y mutated to M at position 497 of SEQ ID NO: 1 and with other amino acid residues unchanged;
QH363, an amino acid sequence with K mutated to A at position 676 and M mutated to I at position 680 of SEQ ID NO: 1 and with other amino acid residues unchanged;
QH329, an amino acid sequence with E mutated to I at position 485 and R mutated to G at position 484 of SEQ ID NO: 1 and with other amino acid residues unchanged;
QH364, an amino acid sequence with K mutated to I at position 676 and M mutated to T at position 680 of SEQ ID NO: 1 and with other amino acid residues unchanged;
QH365, an amino acid sequence with I mutated to K at position 474 and L mutated to V at position 478 of SEQ ID NO: 1 and with other amino acid residues unchanged;
QH370, an amino acid sequence with E mutated to I at position 485 and I mutated to Q at position 666 of SEQ ID NO: 1 and with other amino acid residues unchanged; and
QH371, an amino acid sequence with E mutated to I at position 485 and I mutated to R at position 666 of SEQ ID NO: 1 and with other amino acid residues unchanged.

Specific mutated positions and types of the DNA polymerase mutant with a three-site recombinant mutation based on the KOD DNA polymerase GH78 are as follows:
QH303, an amino acid sequence with K mutated to L at position 676, M mutated to T at position 680, and V mutated to I at position 689 of SEQ ID NO: 1 and with other amino acid residues unchanged;
QH304, an amino acid sequence with L mutated to M at position 457, Q mutated to A at position 461, and T mutated to D at position 267 of SEQ ID NO: 1 and with other amino acid residues unchanged;
QH305, an amino acid sequence with K mutated to Q at position 465, K mutated to T at position 584, and E mutated to I at position 485 of SEQ ID NO: 1 and with other amino acid residues unchanged;
QH306, an amino acid sequence with K mutated to Q at position 465, D mutated to Q at position 718, and E mutated to I at position 485 of SEQ ID NO: 1 and with other amino acid residues unchanged;
QH307, an amino acid sequence with L mutated to M at position 457, Q mutated to A at position 461, and E mutated to I at position 485 of SEQ ID NO: 1 and with other amino acid residues unchanged;
QH308, an amino acid sequence with K mutated to S at position 676, M mutated to I at position 680, and E mutated to I at position 485 of SEQ ID NO: 1 and with other amino acid residues unchanged;
QH309, an amino acid sequence with Y mutated to F at position 493, Y mutated to L at position 497, and E mutated to I at position 485 of SEQ ID NO: 1 and with other amino acid residues unchanged;
QH310, an amino acid sequence with D mutated to N at position 480, S mutated to Q at position 451, and D mutated to Q at position 718 of SEQ ID NO: 1 and with other amino acid residues unchanged;
QH311, an amino acid sequence with D mutated to N at position 480, K mutated to M at position 674, and M mutated to I at position 680 of SEQ ID NO: 1 and with other amino acid residues unchanged;
QH312, an amino acid sequence with D mutated to N at position 480, S mutated to Q at position 451, and E mutated to I at position 485 of SEQ ID NO: 1 and with other amino acid residues unchanged;
QH313, an amino acid sequence with D mutated to N at position 480, K mutated to Q at position 465, and D mutated to Q at position 718 of SEQ ID NO: 1 and with other amino acid residues unchanged; and
QH314, an amino acid sequence with D mutated to N at position 480, S mutated to Q at position 451, and Q mutated to A at position 461 of SEQ ID NO: 1 and with other amino acid residues unchanged.

Specific mutated positions and types of the DNA polymerase mutant with a four-site recombinant mutation based on the KOD DNA polymerase GH78 are as follows:
QH315, an amino acid sequence with K mutated to Q at position 465, D mutated to Q at position 718, L mutated to M at position 457, and Q mutated to A at position 461 of SEQ ID NO: 1 and with other amino acid residues unchanged; and
QH316, an amino acid sequence with K mutated to Q at position 465, D mutated to Q at position 718, D mutated to N at position 480, and E mutated to I at position 485 of SEQ ID NO: 1 and with other amino acid residues unchanged.

Specific mutated positions and types of the DNA polymerase mutant with a three-site recombinant mutation based on the KOD DNA polymerase GH78 are as follows:
QH317, an amino acid sequence with D mutated to N at position 480, S mutated to Q at position 451, and R mutated to Y at position 379 of SEQ ID NO: 1 and with other amino acid residues unchanged;
QH318, an amino acid sequence with L mutated to M at position 457, Q mutated to A at position 461, and K mutated to Q at position 465 of SEQ ID NO: 1 and with other amino acid residues unchanged;
QH319, an amino acid sequence with D mutated to N at position 480, K mutated to Q at position 674, and M mutated to I at position 680 of SEQ ID NO: 1 and with other amino acid residues unchanged;
QH320, an amino acid sequence with D mutated to N at position 480, K mutated to R at position 674, and M mutated to I at position 680 of SEQ ID NO: 1 and with other amino acid residues unchanged;
QH321, an amino acid sequence with D mutated to N at position 480, S mutated to Q at position 451, and K mutated to Q at position 465 of SEQ ID NO: 1 and with other amino acid residues unchanged;
QH322, an amino acid sequence with M mutated to I at position 680, K mutated to H at position 676, and E mutated to I at position 485 of SEQ ID NO: 1 and with other amino acid residues unchanged;
QH323, an amino acid sequence with K mutated to Q at position 465, D mutated to Q at position 718, and T mutated to D at position 267 of SEQ ID NO: 1 and with other amino acid residues unchanged;
QH324, an amino acid sequence with S mutated to Q at position 451, L mutated to V at position 452, and L mutated to V at position 453 of SEQ ID NO: 1 and with other amino acid residues unchanged;
QH325, an amino acid sequence with S mutated to Q at position 451, K mutated to M at position 674, and K mutated to T at position 676 of SEQ ID NO: 1 and with other amino acid residues unchanged;
QH326, an amino acid sequence with K mutated to Q at position 465, D mutated to Q at position 718, and R mutated to Y at position 379 of SEQ ID NO: 1 and with other amino acid residues unchanged;
QH326, an amino acid sequence with K mutated to Q at position 465, D mutated to Q at position 718, and L mutated to V at position 453 of SEQ ID NO: 1 and with other amino acid residues unchanged;
QH328, an amino acid sequence with D mutated to N at position 480, K mutated to I at position 674, and M mutated to I at position 680 of SEQ ID NO: 1 and with other amino acid residues unchanged;
QH331, an amino acid sequence with K mutated to Q at position 465, K mutated to T at position 584, and L mutated to V at position 453 of SEQ ID NO: 1 and with other amino acid residues unchanged;
QH332, an amino acid sequence with K mutated to Q at position 465, D mutated to Q at position 718, and S mutated to G at position 707 of SEQ ID NO: 1 and with other amino acid residues unchanged;
QH333, an amino acid sequence with K mutated to Q at position 465, K mutated to T at position 584, and R mutated to Y at position 379 of SEQ ID NO: 1 and with other amino acid residues unchanged;
QH334, an amino acid sequence with S mutated to Q at position 451, R mutated to K at position 482, and K mutated to T at position 676 of SEQ ID NO: 1 and with other amino acid residues unchanged;
QH335, an amino acid sequence with K mutated to Q at position 465, K mutated to T at position 584, and T mutated to D at position 267 of SEQ ID NO: 1 and with other amino acid residues unchanged; and
QH336, an amino acid sequence with E mutated to I at position 485, K mutated to L at position 676, and M mutated to L at position 680 of SEQ ID NO: 1 and with other amino acid residues unchanged.

Specific mutated positions and types of the DNA polymerase mutant with a four-site recombinant mutation based on the KOD DNA polymerase GH78 are as follows:
QH338, an amino acid sequence with D mutated to N at position 480, T mutated to R at position 667, K mutated to Q at position 674, and M mutated to I at position 680 of SEQ ID NO: 1 and with other amino acid residues unchanged;
QH339, an amino acid sequence with D mutated to N at position 480, T mutated to N at position 667, K mutated to M at position 674, and M mutated to I at position 680 of SEQ ID NO: 1 and with other amino acid residues unchanged;
QH340, an amino acid sequence with D mutated to N at position 480, T mutated to S at position 667, K mutated to M at position 674, and M mutated to I at position 680 of SEQ ID NO: 1 and with other amino acid residues unchanged;
QH341, an amino acid sequence with S mutated to Q at position 451, D mutated to N at position 480, K mutated to Q at position 674, and M mutated to I at position 680 of SEQ ID NO: 1 and with other amino acid residues unchanged;
QH342, an amino acid sequence with E mutated to I at position 485, K mutated to L at position 676, M mutated to T at position 680, and V mutated to I at position 698 of SEQ ID NO: 1 and with other amino acid residues unchanged;
QH343, an amino acid sequence with D mutated to N at position 480, T mutated to N at position 667, K mutated to Q at position 674, and M mutated to I at position 680 of SEQ ID NO: 1 and with other amino acid residues unchanged;
QH344, an amino acid sequence with K mutated to S at position 676, M mutated to I at position 680, Y mutated to F at position 493, and Y mutated to L at position 497 of SEQ ID NO: 1 and with other amino acid residues unchanged; and
QH345, an amino acid sequence with S mutated to Q at position 451, R mutated to K at position 482, K mutated to Q at position 674, and M mutated to L at position 680 of SEQ ID NO: 1 and with other amino acid residues unchanged.

Specific mutated positions and types of the DNA polymerase mutant with a five-site recombinant mutation based on the KOD DNA polymerase GH78 are as follows:
QH346, an amino acid sequence with S mutated to Q at position 451, R mutated to K at position 482, K mutated to M at position 674, M mutated to L at position 680, and L mutated to M at position 479 of SEQ ID NO: 1 and with other amino acid residues unchanged; and
QH347, an amino acid sequence with S mutated to Q at position 451, L mutated to I at position 453, D mutated to N at position 480, K mutated to Q at position 674, and M mutated to I at position 680 of SEQ ID NO: 1 and with other amino acid residues unchanged.

Specific mutated positions and types of the DNA polymerase mutant with a six-site recombinant mutation based on the KOD DNA polymerase GH78 are as follows:
QH348, an amino acid sequence with S mutated to Q at position 451, R mutated to K at position 482, K mutated to Q at position 674, M mutated to L at position 680, L mutated to V at position 452, and D mutated to N at position 480 of SEQ ID NO: 1 and with other amino acid residues unchanged.

The KOD DNA polymerase mutants may be obtained by site-directed mutagenesis based on the KOD DNA polymerase GH78, or by other existing methods.

### 2.1 Construction of Recombinant Vector Expressing the KOD DNA Polymerase Mutant

The recombinant vectors expressing different KOD DNA polymerase mutants individually was obtained by recombining the genes encoding the different KOD DNA polymerase mutants fused with His tag to the pD441-pelB vector individually with operation steps according to the instruction of Electra^{™} Cloning Reagents Kit (DNA2.0, EKT-02). The genes encoding the different KOD DNA polymerase mutants fused with the His tag individually was expressed by guiding by the signal peptide in the pD441-pelB vector.

The amino acid sequences of individual KOD DNA polymerase mutant fusion proteins were obtained by connecting 6× His tag with the N-terminal thereof.

The nucleotide sequences of individual genes encoding the different KOD DNA polymerase mutants fused with His tag were sequences linked with codons of 6×His tag at the 3' end thereof.

### 2.2 Construction of Recombinant Bacteria

According to the steps in 1.2, the recombinant vectors expressing the different KOD DNA polymerase mutants constructed in 2.1 were transformed into *E. coli* BL21 individually, so as to obtain recombinant bacteria expressing the different KOD DNA polymerase mutant fusion proteins individually.

### 3. Expression and Purification of the Mutants

According to the methods of expression and purification for fusion proteins of the wild type KOD DNA polymerase, the different KOD DNA polymerase mutant fusion proteins expressed by the recombinant bacteria individually were expressed and purified so as to obtain the different KOD DNA polymerase mutant fusion proteins.

The different KOD DNA polymerase mutant fusion proteins were subjected to SDS-PAGE (5% stacking gel and 12% separation gel) to obtain target proteins. The purity of each protein after the gel electrophoresis was analyzed with Quantity one software, and the purity of individual KOD DNA polymerase mutant fusion protein reached to 95% or above, after purification.

### Example 2. Properties Tests for Recombinant KOD DNA Polymerase Mutant Fusion Proteins

Detections of the polymerase activity of the recombinant KOD DNA polymerase mutant fusion proteins were performed according to the method disclosed in Nishioka, M., et al. (2001. J. Biotechnol. 88), where one unit of enzyme activity is defined as the amount of acid-insoluble substances generated by polymerizing 10 nmol of dNTP by one unit enzyme in a 50 µl reaction system at 75° C for 30 min.

In this example, dATPs labeled with Cy3 fluorescent dye at 5' terminal (i.e. modified dATPs) and DNA chains labeled with Cy5 fluorescent dye at 5' terminal (i.e. DNA-Cy5) were used, in which the DNA chain was obtained by anneal between a single-stranded DNA as shown in SEQ ID NO: 5 and a single-stranded DNA as shown in SEQ ID NO: 6, and SEQ ID NO: 5 and SEQ ID NO: 6 are partially and reversely complementary for 25 bp. SBS sequencing was simulated with modified nucleotide incorporation, and a relative reaction rate of the recombinant KOD DNA polymerase mutants was detected by using a microplate reader, so as to approximately draw the Michaelis-Menten curve of each mutant and calculate value of Kcat/Km, and the specific experimental method was as follows.

A reaction buffer at pH 8.5 was prepared with 20 mM Tris-HCl, 10 mM (NH₄)₂SO₄, 10 mM KCl, 2 mM MgSO₄ and water.

A reaction system was prepared by mixing 1µg of the KOD DNA polymerase mutant fusion protein, 2 µM of modified dATP, and the DNA-Cy5 with different concentrations between 0.2µM to 0.4µM with the reaction buffer.

The reaction was at 40°C for 1 h.

After the reaction, the data sheet and curve of enzyme activity were exported directly, with which the reaction rate of the relative fluorescence value was approximately calculated and the kinetic curve was fitted according to the Michaelis-Menten equation to calculate values of Vmax and Km.

The KOD DNA polymerase GH78 fusion protein was used as a control.

The kinetic curves of the KOD DNA polymerase mutant fusion proteins are shown in Table 2. It can be seen that the polymerase activity of KOD DNA polymerase mutant is higher than that of GH78, and they show greater potential than GH78. In the sequencing process, the higher the catalytic efficiency of the enzyme reaction, the greater the value of Vmax/Km, and thus the mutants can speed up the reaction to a certain extent and shorten the time required for sequencing reaction. It can be seen from the experimental results that the KOD DNA polymerase mutants KH222, KH223 and KH224 have improved enzyme catalytic efficiencye.

**Table 2 Test results of the kinetics of the KOD DNA polymerase mutants**

| Mutant | Relative Kcat/km | Mutant | Relative Kcat/km | Mutant | Relative Kcat/km |
|---|---|---|---|---|---|
| GH78 | 1 | KH221 | 1.4 | QH303 | 3.3 |
| GH101 | 3.9 | KH288 | 1.8 | QH304 | 3.0 |
| GH102 | 3.0 | KH222 | 31.1 | QH305 | 3.0 |
| GH103 | 2.9 | KH223 | 23.3 | QH306 | 2.9 |
| GH104 | 2.7 | KH224 | 22.8 | QH307 | 2.6 |
| GH105 | 2.3 | KH225 | 14.7 | QH308 | 2.4 |
| GH106 | 2.2 | KH226 | 12.4 | QH309 | 2.4 |
| GH107 | 1.8 | KH227 | 12.1 | QH310 | 2.3 |
| GH108 | 1.8 | KH228 | 10.0 | QH311 | 2.1 |
| GH109 | 1.7 | KH229 | 9.9 | QH312 | 2.0 |
| GH110 | 1.5 | KH230 | 9.7 | QH313 | 2.0 |
| GH111 | 1.4 | KH231 | 9.6 | QH314 | 1.9 |
| GH112 | 1.4 | KH232 | 9.5 | QH315 | 1.8 |
| GH113 | 1.4 | KH233 | 9.4 | QH316 | 1.8 |
| GH114 | 1.4 | KH234 | 8.3 | QH317 | 1.7 |
| GH115 | 1.4 | KH23 5 | 7.6 | QH318 | 1.6 |
| GH116 | 1.3 | KH236 | 7.3 | QH319 | 1.6 |
| GH117 | 1.3 | KH237 | 6.4 | QH320 | 1.6 |
| GH118 | 1.3 | KH23 8 | 6.2 | QH321 | 1.5 |
| GH119 | 1.3 | KH239 | 6.2 | QH322 | 1.5 |
| GH120 | 1.3 | KH240 | 6.1 | QH323 | 1.5 |
| GH121 | 1.3 | KH241 | 6.1 | QH324 | 1.3 |
| GH122 | 1.2 | KH242 | 6.1 | QH325 | 1.3 |
| GH123 | 1.2 | KH243 | 6.0 | QH326 | 1.3 |
| GH124 | 1.2 | KH244 | 6.0 | QH327 | 1.2 |
| GH125 | 1.2 | KH245 | 5.9 | QH328 | 1.2 |
| GH126 | 1.2 | KH246 | 5.4 | QH329 | 1.2 |
| GH127 | 1.1 | KH247 | 5.0 | QH370 | 1.9 |
| GH128 | 1.1 | KH248 | 5.0 | QH331 | 1.2 |
| GH129 | 1.1 | KH249 | 4.8 | QH332 | 1.2 |
| GH131 | 1.1 | KH250 | 4.6 | QH333 | 1.2 |
| GH132 | 1.1 | KH251 | 4.4 | QH334 | 1.1 |
| GH133 | 1.1 | KH252 | 4.2 | QH335 | 1.1 |
| GH134 | 1.1 | KH253 | 3.9 | QH336 | 1.1 |
| GH135 | 1.1 | KH254 | 3.8 | QH337 | 1.0 |
| GH136 | 1.1 | KH255 | 3.3 | QH338 | 2.4 |
| GH137 | 1.1 | KH256 | 3.2 | QH339 | 2.1 |
| GH138 | 1.1 | KH257 | 3.2 | QH340 | 1.9 |
| GH139 | 1.1 | KH258 | 3.1 | QH341 | 1.8 |
| GH140 | 1.1 | KH259 | 3.1 | QH342 | 1.7 |
| GH141 | 1.1 | KH260 | 3.0 | QH343 | 1.6 |
| GH142 | 1.1 | KH261 | 2.9 | QH344 | 1.5 |
| GH143 | 1.1 | KH262 | 2.9 | QH345 | 1.1 |
| GH144 | 1.1 | KH263 | 2.8 | QH346 | 1.1 |
| GH145 | 1.1 | KH264 | 2.8 | QH347 | 1.1 |
| GH146 | 1.05 | KH265 | 2.8 | QH348 | 1.5 |
| GH147 | 1.04 | KH266 | 2.7 | QH349 | 1.3 |
| GH148 | 1.01 | KH267 | 2.6 | QH350 | 1.3 |
| GH149 | 1.01 | KH268 | 2.6 | QH351 | 1.3 |
| KH201 | 1.8 | KH269 | 2.6 | QH352 | 1.3 |
| KH202 | 1.8 | KH270 | 2.6 | QH353 | 1.3 |
| KH203 | 1.7 | KH271 | 2.4 | QH354 | 1.3 |
| KH204 | 1.7 | KH272 | 2.4 | QH355 | 1.3 |
| KH205 | 1.7 | KH273 | 2.4 | QH356 | 1.2 |
| KH206 | 1.7 | KH274 | 2.3 | QH357 | 1.2 |
| KH207 | 1.7 | KH275 | 2.3 | QH358 | 1.2 |
| KH208 | 1.6 | KH276 | 2.3 | QH359 | 1.2 |
| KH209 | 1.6 | KH277 | 2.3 | QH360 | 1.2 |
| KH210 | 1.6 | KH278 | 2.2 | QH361 | 1.2 |
| KH211 | 1.6 | KH279 | 2.2 | QH362 | 1.2 |
| KH212 | 1.6 | KH280 | 2.2 | QH363 | 1.2 |
| KH213 | 1.5 | KH281 | 2.1 | QH364 | 1.1 |
| KH214 | 1.5 | KH282 | 2.1 | QH365 | 1.1 |
| KH215 | 1.4 | KH283 | 2.1 | QH366 | 1.1 |
| KH216 | 1.4 | KH284 | 2.0 | QH367 | 1.1 |
| KH217 | 1.4 | KH285 | 2.0 | QH368 | 1.1 |
| KH218 | 1.4 | KH286 | 1.9 | QH371 | 1.6 |
| KH219 | 1.4 | KH287 | 1.9 | | |
| KH220 | 1.4 | QH369 | 2.3 | | |

The relative Kcat/km is calculated by comparing the value of Kcat/km of the mutant with that of GH78, the higher the ratio between the two, the faster the enzyme converting the substrate.

### INDUSTRIAL APPLICATION

The experiment of the present disclosure proves that aiming at further extending the read length of sequencing, shortening the reaction time and other improvement goals, the present disclosure further modified the KOD DNA polymerase based on the previous work, in which original functional sites of B-family DNA polymerase related are protected, thereby ensuring the polymerase still performing its originally basic functions while improving the reaction rate of the DNA polymerase and the length of reads in the sequencing. Compared with the KOD DNA polymerase GH78, the recombinant DNA polymerases provided in embodiments of the present disclosure exhibit better reaction rate, higher catalytic efficiency, better affinity and other advantages in aspect of catalysis, thereby improving the reaction rate of the DNA polymerase and the length of reads in the sequencing.

## Claims

1. A protein having DNA polymerase activity, wherein
the protein has an amino acid sequence with modification on one or more of amino acid residue selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on a KOD DNA polymerase GH78, and with other amino acid residues unchanged,
the KOD DNA polymerase GH78 is set forth in SEQ ID NO: 1,
optionally, the protein is further added with a first tag sequence at the terminal of the protein.

2. The protein of claim 1, wherein the protein has the amino acid sequence with the modification on at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from the group consisting of 48 positions at 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729 based on the KOD DNA polymerase GH78, and with other amino acid residues unchanged.

3. The protein of claim 1 or 2, wherein the modification is substitution of an amino acid.

4. The protein of any one of claims 1 to 3, wherein the substitution of individual amino acids are as follows:
T at position 267 is substituted with D;
F at position 326 is substituted with L;
S at position 347 is substituted with D or V;
T at position 349 is substituted with G;
V at position 353 is substituted with D;
K at position 375 is substituted with I, A or E;
A at position 378 is substituted with E, Q or K;
R at position 379 is substituted with K, Q, E, H or Y;
R at position 380 is substituted with E, D, S, G, N or Y;
E at position 385 is substituted with F or R;
S at position 451 is substituted with N, T, G, H, M, L, P, R, A, Q or K;
L at position 452 is substituted with V;
L at position 453 is substituted with A, T, V or I;
G at position 454 is substituted with Q;
L at position 457 is substituted with M;
Q at position 461 is substituted with A or S;
K at position 465 is substituted with Q;
T at position 470 is substituted with S;
L at position 474 is substituted with K, V, A or T;
K at position 477 is substituted with E;
L at position 478 is mutated to I, V, Y, M, C, N, H or G;
L at position 479 is mutated to M;
D at position 480 is mutated to S, R, T, N, G, F, H or E;
R at position 482 is mutated to K;
R at position 484 is mutated to C, D, I, V, K or G;
E at position 485 is mutated to I, C or F;
I at position 486 is mutated to K or R;
Y at position 493 is mutated to F, H or L;
Y at position 496 is mutated to T or M;
Y at position 497 is mutated to L, F, M, I or W;
T at position 514 was mutated to A;
A at position 574 is mutated to L;
K at position 584 was mutated to T;
T at position 605 is mutated to W or V;
I at position 610 is mutated to L or M;
L at position 630 is mutated to F;
a glutamic acid at position 665 is mutated to R, S, K or C;
an asparagine at position 666 is mutated to A, N, Q, T or K;
T at position 667 is mutated to R, N or S;
K at position 674 is mutated to Q, M, I, E or R;
K at position 676 is mutated to S, L, R, H, V, I, M, A or T;
M at position 680 is mutated to I, L, T or A;
V at position 682 is mutated to M;
V at position 698 is mutated to I;
S at position 707 is mutated to G, T or K;
D at position 718 is mutated to Q;
T at position 723 is mutated to V, C, G, I or F;
A at position 729 is mutated to T.

5. The protein of any one of claims 1 to 4, wherein the protein is any one of the following KOD DNA polymerase mutants, with an identifier and mutated position(s) thereof shown as follows:
GH101, an amino acid sequence with D at position 480 of SEQ ID NO: 1 mutated to N and with other amino acid residues unchanged;
GH102, an amino acid sequence with K at position 676 of SEQ ID NO: 1 mutated to H and with other amino acid residues unchanged;
GH103, an amino acid sequence with E at position 485 of SEQ ID NO: 1 mutated to I and with other amino acid residues unchanged;
GH104, an amino acid sequence with Y at position 497 of SEQ ID NO: 1 mutated to L and with other amino acid residues unchanged;
GH105, an amino acid sequence with S at position 451 of SEQ ID NO: 1 mutated to Q and with other amino acid residues unchanged;
GH106, an amino acid sequence with R at position 482 of SEQ ID NO: 1 mutated to K and with other amino acid residues unchanged;
GH107, an amino acid sequence with L at position 453 of SEQ ID NO: 1 mutated to V and with other amino acid residues unchanged;
GH108, an amino acid sequence with I at position 474 of SEQ ID NO: 1 mutated to T and with other amino acid residues unchanged;
GH109, an amino acid sequence with T at position 267 of SEQ ID NO: 1 mutated to D and with other amino acid residues unchanged;
GH110, an amino acid sequence with T at position 723 of SEQ ID NO: 1 mutated to V and with other amino acid residues unchanged;
GH111, an amino acid sequence with R at position 379 of SEQ ID NO: 1 mutated to Y and with other amino acid residues unchanged;
GH112, an amino acid sequence with L at position 453 of SEQ ID NO: 1 mutated to I and with other amino acid residues unchanged;
GH113, an amino acid sequence with I at position 474 of SEQ ID NO: 1 mutated to K and with other amino acid residues unchanged;
GH114, an amino acid sequence with V at position 353 of SEQ ID NO: 1 mutated to D and with other amino acid residues unchanged;
GH115, an amino acid sequence with S at position 347 of SEQ ID NO: 1 mutated to D and with other amino acid residues unchanged;
GH116, an amino acid sequence with K at position 465 of SEQ ID NO: 1 mutated to Q and with other amino acid residues unchanged;
GH117, an amino acid sequence with R at position 380 of SEQ ID NO: 1 mutated to D and with other amino acid residues unchanged;
GH118, an amino acid sequence with Q at position 461 of SEQ ID NO: 1 mutated to A and with other amino acid residues unchanged;
GH119, an amino acid sequence with I at position 610 of SEQ ID NO: 1 mutated to L and with other amino acid residues unchanged;
GH120, an amino acid sequence with T at position 723 of SEQ ID NO: 1 mutated to G and with other amino acid residues unchanged;
GH121, an amino acid sequence with Y at position 496 of SEQ ID NO: 1 mutated to M and with other amino acid residues unchanged;
GH122, an amino acid sequence with K at position 375 of SEQ ID NO: 1 mutated to A and with other amino acid residues unchanged;
GH123, an amino acid sequence with Y at position 497 of SEQ ID NO: 1 mutated to I and with other amino acid residues unchanged;
GH124, an amino acid sequence with I at position 474 of SEQ ID NO: 1 mutated to A and with other amino acid residues unchanged;
GH125, an amino acid sequence with S at position 707 of SEQ ID NO: 1 mutated to G and with other amino acid residues unchanged;
GH126, an amino acid sequence with R at position 379 of SEQ ID NO: 1 mutated to E and with other amino acid residues unchanged;
GH127, an amino acid sequence with T at position 723 of SEQ ID NO: 1 mutated to I and with other amino acid residues unchanged;
GH128, an amino acid sequence with Q at position 461 of SEQ ID NO: 1 mutated to S and with other amino acid residues unchanged;
GH129, an amino acid sequence with D at position 718 of SEQ ID NO: 1 mutated to Q and with other amino acid residues unchanged;
GH131, an amino acid sequence with L at position 452 of SEQ ID NO: 1 mutated to V and with other amino acid residues unchanged;
GH132, an amino acid sequence with R at position 380 of SEQ ID NO: 1 mutated to Y and with other amino acid residues unchanged;
GH133, an amino acid sequence with L at position 478 of SEQ ID NO: 1 mutated to V and with other amino acid residues unchanged;
GH134, an amino acid sequence with L at position 478 of SEQ ID NO: 1 mutated to Y and with other amino acid residues unchanged;
GH135, an amino acid sequence with E at position 385 of SEQ ID NO: 1 mutated to R and with other amino acid residues unchanged;
GH136, an amino acid sequence with I at position 474 of SEQ ID NO: 1 mutated to V and with other amino acid residues unchanged;
GH137, an amino acid sequence with L at position 479 of SEQ ID NO: 1 mutated to M and with other amino acid residues unchanged;
GH138, an amino acid sequence with M at position 680 of SEQ ID NO: 1 mutated to L and with other amino acid residues unchanged;
GH139, an amino acid sequence with L at position 478 of SEQ ID NO: 1 mutated to I and with other amino acid residues unchanged;
GH140, an amino acid sequence with T at position 723 of SEQ ID NO: 1 mutated to C and with other amino acid residues unchanged;
GH141, an amino acid sequence with L at position 478 of SEQ ID NO: 1 mutated to M and with other amino acid residues unchanged;
GH142, an amino acid sequence with A at position 729 of SEQ ID NO: 1 mutated to T and with other amino acid residues unchanged;
GH143, an amino acid sequence with I at position 610 of SEQ ID NO: 1 mutated to M and with other amino acid residues unchanged;
GH144, an amino acid sequence with V at position 353 of SEQ ID NO: 1 mutated to D and with other amino acid residues unchanged;
GH145, an amino acid sequence with K at position 676 of SEQ ID NO: 1 mutated to S and with other amino acid residues unchanged;
GH146, an amino acid sequence with F at position 326 of SEQ ID NO: 1 mutated to L and with other amino acid residues unchanged;
GH147, an amino acid sequence with A at position 574 of SEQ ID NO: 1 mutated to L and with other amino acid residues unchanged;
GH148, an amino acid sequence with L at position 630 of SEQ ID NO: 1 mutated to F and with other amino acid residues unchanged;
GH149, an amino acid sequence with T at position 349 of SEQ ID NO: 1 mutated to G and with other amino acid residues unchanged;
KH222, an amino acid sequence with E mutated to I at position 485 and S mutated to L at position 451 of SEQ ID NO: 1 and with other amino acid residues unchanged;
KH223, an amino acid sequence with E mutated to I at position 485 and S mutated to T at position 451 of SEQ ID NO: 1 and with other amino acid residues unchanged;
KH224, an amino acid sequence with E mutated to I at position 485 and D mutated to C at position 484 of SEQ ID NO: 1 and with other amino acid residues unchanged;
KH225, an amino acid sequence with E mutated to I at position 485 and D mutated to T at position 480 of SEQ ID NO: 1 and with other amino acid residues unchanged;
KH226, an amino acid sequence with E mutated to I at position 485 and S mutated to G at position 451 of SEQ ID NO: 1 and with other amino acid residues unchanged;
KH227, an amino acid sequence with E mutated to I at position 485 and L mutated to A at position 453 of SEQ ID NO: 1 and with other amino acid residues unchanged;
KH228, an amino acid sequence with E mutated to C at position 485 and R mutated to V at position 484 of SEQ ID NO: 1 and with other amino acid residues unchanged;
KH229, an amino acid sequence with E mutated to C at position 485 and S mutated to N at position 451 of SEQ ID NO: 1 and with other amino acid residues unchanged;
KH230, an amino acid sequence with E mutated to C at position 485 and D mutated to N at position 480 of SEQ ID NO: 1 and with other amino acid residues unchanged;
KH232, an amino acid sequence with E mutated to C at position 485 and R mutated to I at position 484 of SEQ ID NO: 1 and with other amino acid residues unchanged;
KH233, an amino acid sequence with K mutated to S at position 676 and M mutated to I at position 680 of SEQ ID NO: 1 and with other amino acid residues unchanged;
KH234, an amino acid sequence with E mutated to C at position 485 and I mutated to R at position 486 of SEQ ID NO: 1 and with other amino acid residues unchanged;
KH235, an amino acid sequence with E mutated to I at position 485 and D mutated to S at position 480 of SEQ ID NO: 1 and with other amino acid residues unchanged;
KH236, an amino acid sequence with Y mutated to F at position 493 and Y mutated to L at position 497 of SEQ ID NO: 1 and with other amino acid residues unchanged;
KH237, an amino acid sequence with K mutated to L at position 676 and M mutated to I at position 680 of SEQ ID NO: 1 and with other amino acid residues unchanged;
KH238, an amino acid sequence with E mutated to I at position 485 and D mutated to Q at position 480 of SEQ ID NO: 1 and with other amino acid residues unchanged;
KH239, an amino acid sequence with E mutated to I at position 485 and D mutated to M at position 480 of SEQ ID NO: 1 and with other amino acid residues unchanged;
KH240, an amino acid sequence with E mutated to I at position 485 and S mutated to K at position 451 of SEQ ID NO: 1 and with other amino acid residues unchanged;
KH241, an amino acid sequence with E mutated to I at position 485 and D mutated to R at position 480 of SEQ ID NO: 1 and with other amino acid residues unchanged;
KH242, an amino acid sequence with E mutated to I at position 485 and L mutated to N at position 453 of SEQ ID NO: 1 and with other amino acid residues unchanged;
KH243, an amino acid sequence with K mutated to R at position 676 and M mutated to I at position 680 of SEQ ID NO: 1 and with other amino acid residues unchanged;
KH244, an amino acid sequence with E mutated to I at position 485 and S mutated to M at position 451 of SEQ ID NO: 1 and with other amino acid residues unchanged;
KH245, an amino acid sequence with E mutated to I at position 485 and K mutated to E at position 477 of SEQ ID NO: 1 and with other amino acid residues unchanged;
KH246, an amino acid sequence with E mutated to I at position 485 and D mutated to W at position 480 of SEQ ID NO: 1 and with other amino acid residues unchanged;
KH247, an amino acid sequence with E mutated to I at position 485 and D mutated to K at position 480 of SEQ ID NO: 1 and with other amino acid residues unchanged;
KH248, an amino acid sequence with E mutated to I at position 485 and S mutated to R at position 451 of SEQ ID NO: 1 and with other amino acid residues unchanged;
KH249, an amino acid sequence with K mutated to L at position 676 and M mutated to L at position 680 of SEQ ID NO: 1 and with other amino acid residues unchanged;
KH250, an amino acid sequence with E mutated to I at position 485 and Q mutated to R at position 665 of SEQ ID NO: 1 and with other amino acid residues unchanged;
KH251, an amino acid sequence with E mutated to I at position 485 and Q mutated to S at position 665 of SEQ ID NO: 1 and with other amino acid residues unchanged;
KH252, an amino acid sequence with E mutated to I at position 485 and D mutated to E at position 480 of SEQ ID NO: 1 and with other amino acid residues unchanged;
KH253, an amino acid sequence with K mutated to H at position 676 and M mutated to I at position 680 of SEQ ID NO: 1 and with other amino acid residues unchanged;
KH254, an amino acid sequence with E mutated to I at position 485 and Q mutated to A at position 461 of SEQ ID NO: 1 and with other amino acid residues unchanged;
KH255, an amino acid sequence with E mutated to I at position 485 and S mutated to A at position 451 of SEQ ID NO: 1 and with other amino acid residues unchanged;
KH256, an amino acid sequence with E mutated to I at position 485 and S mutated to Q at position 451 of SEQ ID NO: 1 and with other amino acid residues unchanged;
KH257, an amino acid sequence with E mutated to I at position 485 and I mutated to Q at position 666 of SEQ ID NO: 1 and with other amino acid residues unchanged;
KH258, an amino acid sequence with E mutated to I at position 485 and I mutated to T at position 666 of SEQ ID NO: 1 and with other amino acid residues unchanged;
KH259, an amino acid sequence with E mutated to I at position 485 and T mutated to W at position 605 of SEQ ID NO: 1 and with other amino acid residues unchanged;
KH260, an amino acid sequence with E mutated to I at position 485 and R mutated to Y at position 379 of SEQ ID NO: 1 and with other amino acid residues unchanged;
KH261, an amino acid sequence with E mutated to I at position 485 and L mutated to T at position 453 of SEQ ID NO: 1 and with other amino acid residues unchanged;
KH262, an amino acid sequence with E mutated to I at position 485 and D mutated to C at position 480 of SEQ ID NO: 1 and with other amino acid residues unchanged;
KH263, an amino acid sequence with E mutated to I at position 485 and T mutated to V at position 605 of SEQ ID NO: 1 and with other amino acid residues unchanged;
KH264, an amino acid sequence with E mutated to I at position 485 and D mutated to N at position 480 of SEQ ID NO: 1 and with other amino acid residues unchanged;
KH266, an amino acid sequence with E mutated to I at position 485 and D mutated to G at position 480 of SEQ ID NO: 1 and with other amino acid residues unchanged;
KH267, an amino acid sequence with K mutated to Q at position 465 and D mutated to Q at position 718 of SEQ ID NO: 1 and with other amino acid residues unchanged;
KH268, an amino acid sequence with E mutated to I at position 485 and R mutated to H at position 379 of SEQ ID NO: 1 and with other amino acid residues unchanged;
KH269, an amino acid sequence with E mutated to I at position 485 and K mutated to Q at position 465 of SEQ ID NO: 1 and with other amino acid residues unchanged;
KH270, an amino acid sequence with E mutated to I at position 485 and D mutated to P at position 480 of SEQ ID NO: 1 and with other amino acid residues unchanged;
KH271, an amino acid sequence with E mutated to I at position 485 and D mutated to F at position 480 of SEQ ID NO: 1 and with other amino acid residues unchanged;
KH272, an amino acid sequence with K mutated to V at position 676 and M mutated to L at position 680 of SEQ ID NO: 1 and with other amino acid residues unchanged;
KH273, an amino acid sequence with E mutated to I at position 485 and L mutated to V at position 453 of SEQ ID NO: 1 and with other amino acid residues unchanged;
KH274, an amino acid sequence with E mutated to I at position 485 and D mutated to H at position 480 of SEQ ID NO: 1 and with other amino acid residues unchanged;
KH275, an amino acid sequence with E mutated to I at position 485 and S mutated to P at position 451 of SEQ ID NO: 1 and with other amino acid residues unchanged;
KH276, an amino acid sequence with E mutated to I at position 485 and Q mutated to K at position 665 of SEQ ID NO: 1 and with other amino acid residues unchanged;
KH277, an amino acid sequence with D mutated to S at position 480 and S mutated to Q at position 451 of SEQ ID NO: 1 and with other amino acid residues unchanged;
KH278, an amino acid sequence with I mutated to T at position 474 and L mutated to C at position 478 of SEQ ID NO: 1 and with other amino acid residues unchanged;
KH279, an amino acid sequence with K mutated to H at position 676 and M mutated to T at position 680 of SEQ ID NO: 1 and with other amino acid residues unchanged;
KH280, an amino acid sequence with E mutated to I at position 485 and S mutated to V at position 347 of SEQ ID NO: 1 and with other amino acid residues unchanged;
KH281, an amino acid sequence with D mutated to N at position 480 and L mutated to V at position 453 of SEQ ID NO: 1 and with other amino acid residues unchanged;
KH282, an amino acid sequence with E mutated to I at position 485 and I mutated to A at position 666 of SEQ ID NO: 1 and with other amino acid residues unchanged;
KH283, an amino acid sequence with K mutated to V at position 676 and M mutated to I at position 680 of SEQ ID NO: 1 and with other amino acid residues unchanged;
KH284, an amino acid sequence with K mutated to I at position 676 and M mutated to L at position 680 of SEQ ID NO: 1 and with other amino acid residues unchanged;
KH285, an amino acid sequence with K mutated to M at position 676 and M mutated to A at position 680 of SEQ ID NO: 1 and with other amino acid residues unchanged;
KH286, an amino acid sequence with E mutated to I at position 485 and T mutated to S at position 470 of SEQ ID NO: 1 and with other amino acid residues unchanged;
KH287, an amino acid sequence with K mutated to L at position 676 and M mutated to A at position 680 of SEQ ID NO: 1 and with other amino acid residues unchanged;
KH288, an amino acid sequence with R mutated to K at position 379 and R mutated to E at position 380 of SEQ ID NO: 1 and with other amino acid residues unchanged;
KH201, an amino acid sequence with D mutated to N at position 480 and M mutated to I at position 680 of SEQ ID NO: 1 and with other amino acid residues unchanged;
KH202, an amino acid sequence with I mutated to T at position 474 and L mutated to N at position 478 of SEQ ID NO: 1 and with other amino acid residues unchanged;
KH203, an amino acid sequence with I mutated to T at position 474 and L mutated to H at position 478 of SEQ ID NO: 1 and with other amino acid residues unchanged;
KH204, an amino acid sequence with E mutated to I at position 485 and R mutated to K at position 484 of SEQ ID NO: 1 and with other amino acid residues unchanged;
KH205, an amino acid sequence with Y mutated to H at position 493 and Y mutated to F at position 497 of SEQ ID NO: 1 and with other amino acid residues unchanged;
KH206, an amino acid sequence with Y mutated to F at position 493 and Y mutated to M at position 497 of SEQ ID NO: 1 and with other amino acid residues unchanged;
KH207, an amino acid sequence with T mutated to A at position 514 and K mutated to T at position 584 of SEQ ID NO: 1 and with other amino acid residues unchanged;
KH208, an amino acid sequence with I mutated to T at position 474 and L mutated to G at position 478 of SEQ ID NO: 1 and with other amino acid residues unchanged;
KH209, an amino acid sequence with E mutated to I at position 485 and Q mutated to C at position 665 of SEQ ID NO: 1 and with other amino acid residues unchanged;
KH210, an amino acid sequence with K mutated to Q at position 465 and K mutated to T at position 584 of SEQ ID NO: 1 and with other amino acid residues unchanged;
KH211, an amino acid sequence with K mutated to M at position 676 and M mutated to L at position 680 of SEQ ID NO: 1 and with other amino acid residues unchanged;
KH212, an amino acid sequence with Y mutated to H at position 493 and Y mutated to L at position 497 of SEQ ID NO: 1 and with other amino acid residues unchanged;
KH213, an amino acid sequence with L mutated to M at position 457 and Q mutated to A at position 461 of SEQ ID NO: 1 and with other amino acid residues unchanged;
KH214, an amino acid sequence with K mutated to S at position 676 and M mutated to A at position 680 of SEQ ID NO: 1 and with other amino acid residues unchanged;
KH125, an amino acid sequence with E mutated to F at position 485 and I mutated to K at position 486 of SEQ ID NO: 1 and with other amino acid residues unchanged;
KH126, an amino acid sequence with D mutated to N at position 480 and K mutated to T at position 584 of SEQ ID NO: 1 and with other amino acid residues unchanged;
KH217, an amino acid sequence with K mutated to E at position 375 and A mutated to K at position 378 of SEQ ID NO: 1 and with other amino acid residues unchanged;
KH218, an amino acid sequence with Y mutated to F at position 493 and Y mutated to I at position 497 of SEQ ID NO: 1 and with other amino acid residues unchanged;
KH219, an amino acid sequence with I mutated to K at position 474 and L mutated to H at position 478 of SEQ ID NO: 1 and with other amino acid residues unchanged;
KH220, an amino acid sequence with V mutated to D at position 353 and Y mutated to T at position 496 of SEQ ID NO: 1 and with other amino acid residues unchanged;
KH221, an amino acid sequence with R mutated to Q at position 379 and R mutated to D at position 380 of SEQ ID NO: 1 and with other amino acid residues unchanged;
QH349, an amino acid sequence with K mutated to A at position 676 and M mutated to L at position 680 of SEQ ID NO: 1 and with other amino acid residues unchanged;
QH350, an amino acid sequence with D mutated to N at position 480 and R mutated to K at position 482 of SEQ ID NO: 1 and with other amino acid residues unchanged;
QH351, an amino acid sequence with S mutated to Q at position 451 and L mutated to V at position 453 of SEQ ID NO: 1 and with other amino acid residues unchanged;
QH352, an amino acid sequence with I mutated to K at position 474 and L mutated to Y at position 478 of SEQ ID NO: 1 and with other amino acid residues unchanged;
QH353, an amino acid sequence with D mutated to N at position 480 and T mutated to D at position 267 of SEQ ID NO: 1 and with other amino acid residues unchanged;
QH354, an amino acid sequence with K mutated to L at position 676 and M mutated to T at position 680 of SEQ ID NO: 1 and with other amino acid residues unchanged;
QH355, an amino acid sequence with E mutated to I at position 485 and G mutated to Q at position 454 of SEQ ID NO: 1 and with other amino acid residues unchanged;
QH356, an amino acid sequence with K mutated to V at position 676 and V mutated to M at position 682 of SEQ ID NO: 1 and with other amino acid residues unchanged;
QH357, an amino acid sequence with K mutated to Q at position 465 and S mutated to G at position 707 of SEQ ID NO: 1 and with other amino acid residues unchanged;
QH358, an amino acid sequence with K mutated to I at position 676 and M mutated to I at position 680 of SEQ ID NO: 1 and with other amino acid residues unchanged;
QH359, an amino acid sequence with I mutated to K at position 474 and L mutated to G at position 478 of SEQ ID NO: 1 and with other amino acid residues unchanged;
QH360, an amino acid sequence with R mutated to E at position 379 and R mutated to S at position 380 of SEQ ID NO: 1 and with other amino acid residues unchanged;
QH361, an amino acid sequence with K mutated to I at position 375 and A mutated to Q at position 378 of SEQ ID NO: 1 and with other amino acid residues unchanged;
QH362, an amino acid sequence with Y mutated to H at position 493 and Y mutated to M at position 497 of SEQ ID NO: 1 and with other amino acid residues unchanged;
QH363, an amino acid sequence with K mutated to A at position 676 and M mutated to I at position 680 of SEQ ID NO: 1 and with other amino acid residues unchanged;
QH329, an amino acid sequence with E mutated to I at position 485 and R mutated to G at position 484 of SEQ ID NO: 1 and with other amino acid residues unchanged;
QH364, an amino acid sequence with K mutated to I at position 676 and M mutated to T at position 680 of SEQ ID NO: 1 and with other amino acid residues unchanged;
QH365, an amino acid sequence with I mutated to K at position 474 and L mutated to V at position 478 of SEQ ID NO: 1 and with other amino acid residues unchanged;
QH366, an amino acid sequence with R mutated to K at position 379 and R mutated to G at position 380 of SEQ ID NO: 1 and with other amino acid residues unchanged;
QH367, an amino acid sequence with R mutated to Q at position 379 and R mutated to S at position 380 of SEQ ID NO: 1 and with other amino acid residues unchanged;
QH368, an amino acid sequence with R mutated to Q at position 379 and R mutated to N at position 380 of SEQ ID NO: 1 and with other amino acid residues unchanged;
QH369, an amino acid sequence with E mutated to I at position 485 and S mutated to Q at position 451 of SEQ ID NO: 1 and with other amino acid residues unchanged;
QH370, an amino acid sequence with E mutated to I at position 485 and I mutated to Q at position 666 of SEQ ID NO: 1 and with other amino acid residues unchanged;
QH371, an amino acid sequence with E mutated to I at position 485 and I mutated to R at position 666 of SEQ ID NO: 1 and with other amino acid residues unchanged;
QH303, an amino acid sequence with K mutated to L at position 676, M mutated to T at position 680, and V mutated to I at position 689 of SEQ ID NO: 1 and with other amino acid residues unchanged;
QH304, an amino acid sequence with L mutated to M at position 457, Q mutated to A at position 461, and T mutated to D at position 267 of SEQ ID NO: 1 and with other amino acid residues unchanged;
QH305, an amino acid sequence with K mutated to Q at position 465, K mutated to T at position 584, and E mutated to I at position 485 of SEQ ID NO: 1 and with other amino acid residues unchanged;
QH306, an amino acid sequence with K mutated to Q at position 465, D mutated to Q at position 718, and E mutated to I at position 485 of SEQ ID NO: 1 and with other amino acid residues unchanged;
QH307, an amino acid sequence with L mutated to M at position 457, Q mutated to A at position 461, and E mutated to I at position 485 of SEQ ID NO: 1 and with other amino acid residues unchanged;
QH308, an amino acid sequence with K mutated to S at position 676, M mutated to I at position 680, and E mutated to I at position 485 of SEQ ID NO: 1 and with other amino acid residues unchanged;
QH309, an amino acid sequence with Y mutated to F at position 493, Y mutated to L at position 497, and E mutated to I at position 485 of SEQ ID NO: 1 and with other amino acid residues unchanged;
QH310, an amino acid sequence with D mutated to N at position 480, S mutated to Q at position 451, and D mutated to Q at position 718 of SEQ ID NO: 1 and with other amino acid residues unchanged;
QH311, an amino acid sequence with D mutated to N at position 480, K mutated to M at position 674, and M mutated to I at position 680 of SEQ ID NO: 1 and with other amino acid residues unchanged;
QH312, an amino acid sequence with D mutated to N at position 480, S mutated to Q at position 451, and E mutated to I at position 485 of SEQ ID NO: 1 and with other amino acid residues unchanged;
QH313, an amino acid sequence with D mutated to N at position 480, K mutated to Q at position 465, and D mutated to Q at position 718 of SEQ ID NO: 1 and with other amino acid residues unchanged;
QH314, an amino acid sequence with D mutated to N at position 480, S mutated to Q at position 451, and Q mutated to A at position 461 of SEQ ID NO: 1 and with other amino acid residues unchanged;
QH315, an amino acid sequence with K mutated to Q at position 465, D mutated to Q at position 718, L mutated to M at position 457, and Q mutated to A at position 461 of SEQ ID NO: 1 and with other amino acid residues unchanged;
QH316, an amino acid sequence with K mutated to Q at position 465, D mutated to Q at position 718, D mutated to N at position 480, and E mutated to I at position 485 of SEQ ID NO: 1 and with other amino acid residues unchanged;
QH317, an amino acid sequence with D mutated to N at position 480, S mutated to Q at position 451, and R mutated to Y at position 379 of SEQ ID NO: 1 and with other amino acid residues unchanged;
QH318, an amino acid sequence with L mutated to M at position 457, Q mutated to A at position 461, and K mutated to Q at position 465 of SEQ ID NO: 1 and with other amino acid residues unchanged;
QH319, an amino acid sequence with D mutated to N at position 480, K mutated to Q at position 674, and M mutated to I at position 680 of SEQ ID NO: 1 and with other amino acid residues unchanged;
QH320, an amino acid sequence with D mutated to N at position 480, K mutated to R at position 674, and M mutated to I at position 680 of SEQ ID NO: 1 and with other amino acid residues unchanged;
QH321, an amino acid sequence with D mutated to N at position 480, S mutated to Q at position 451, and K mutated to Q at position 465 of SEQ ID NO: 1 and with other amino acid residues unchanged;
QH322, an amino acid sequence with M mutated to I at position 680, K mutated to H at position 676, and E mutated to I at position 485 of SEQ ID NO: 1 and with other amino acid residues unchanged;
QH323, an amino acid sequence with K mutated to Q at position 465, D mutated to Q at position 718, and T mutated to D at position 267 of SEQ ID NO: 1 and with other amino acid residues unchanged;
QH324, an amino acid sequence with S mutated to Q at position 451, L mutated to V at position 452, and L mutated to V at position 453 of SEQ ID NO: 1 and with other amino acid residues unchanged;
QH325, an amino acid sequence with S mutated to Q at position 451, K mutated to M at position 674, and K mutated to T at position 676 of SEQ ID NO: 1 and with other amino acid residues unchanged;
QH326, an amino acid sequence with K mutated to Q at position 465, D mutated to Q at position 718, and R mutated to Y at position 379 of SEQ ID NO: 1 and with other amino acid residues unchanged;
QH326, an amino acid sequence with K mutated to Q at position 465, D mutated to Q at position 718, and L mutated to V at position 453 of SEQ ID NO: 1 and with other amino acid residues unchanged;
QH328, an amino acid sequence with D mutated to N at position 480, K mutated to I at position 674, and M mutated to I at position 680 of SEQ ID NO: 1 and with other amino acid residues unchanged;
QH331, an amino acid sequence with K mutated to Q at position 465, K mutated to T at position 584, and L mutated to V at position 453 of SEQ ID NO: 1 and with other amino acid residues unchanged;
QH332, an amino acid sequence with K mutated to Q at position 465, D mutated to Q at position 718, and S mutated to G at position 707 of SEQ ID NO: 1 and with other amino acid residues unchanged;
QH333, an amino acid sequence with K mutated to Q at position 465, K mutated to T at position 584, and R mutated to Y at position 379 of SEQ ID NO: 1 and with other amino acid residues unchanged;
QH334, an amino acid sequence with S mutated to Q at position 451, R mutated to K at position 482, and K mutated to T at position 676 of SEQ ID NO: 1 and with other amino acid residues unchanged;
QH335, an amino acid sequence with K mutated to Q at position 465, K mutated to T at position 584, and T mutated to D at position 267 of SEQ ID NO: 1 and with other amino acid residues unchanged;
QH336, an amino acid sequence with E mutated to I at position 485, K mutated to L at position 676, and M mutated to L at position 680 of SEQ ID NO: 1 and with other amino acid residues unchanged;
QH338, an amino acid sequence with D mutated to N at position 480, T mutated to R at position 667, K mutated to Q at position 674, and M mutated to I at position 680 of SEQ ID NO: 1 and with other amino acid residues unchanged;
QH339, an amino acid sequence with D mutated to N at position 480, T mutated to N at position 667, K mutated to M at position 674, and M mutated to I at position 680 of SEQ ID NO: 1 and with other amino acid residues unchanged;
QH340, an amino acid sequence with D mutated to N at position 480, T mutated to S at position 667, K mutated to M at position 674, and M mutated to I at position 680 of SEQ ID NO: 1 and with other amino acid residues unchanged;
QH341, an amino acid sequence with S mutated to Q at position 451, D mutated to N at position 480, K mutated to Q at position 674, and M mutated to I at position 680 of SEQ ID NO: 1 and with other amino acid residues unchanged;
QH342, an amino acid sequence with E mutated to I at position 485, K mutated to L at position 676, M mutated to T at position 680, and V mutated to I at position 698 of SEQ ID NO: 1 and with other amino acid residues unchanged;
QH343, an amino acid sequence with D mutated to N at position 480, T mutated to N at position 667, K mutated to Q at position 674, and M mutated to I at position 680 of SEQ ID NO: 1 and with other amino acid residues unchanged;
QH344, an amino acid sequence with K mutated to S at position 676, M mutated to I at position 680, Y mutated to F at position 493, and Y mutated to L at position 497 of SEQ ID NO: 1 and with other amino acid residues unchanged;
QH345, an amino acid sequence with S mutated to Q at position 451, R mutated to K at position 482, K mutated to Q at position 674, and M mutated to L at position 680 of SEQ ID NO: 1 and with other amino acid residues unchanged;
QH346, an amino acid sequence with S mutated to Q at position 451, R mutated to K at position 482, K mutated to M at position 674, M mutated to L at position 680, and L mutated to M at position 479 of SEQ ID NO: 1 and with other amino acid residues unchanged;
QH347, an amino acid sequence with S mutated to Q at position 451, L mutated to I at position 453, D mutated to N at position 480, K mutated to Q at position 674, and M mutated to I at position 680 of SEQ ID NO: 1 and with other amino acid residues unchanged; and
QH348, an amino acid sequence with S mutated to Q at position 451, R mutated to K at position 482, K mutated to Q at position 674, M mutated to L at position 680, L mutated to V at position 452, and D mutated to N at position 480 of SEQ ID NO: 1 and with other amino acid residues unchanged.

6. A DNA molecule encoding a protein of any one of claims 1 to 5.

7. The DNA molecule of claim 6, wherein the DNA molecule has a first nucleotide sequence encoding a mutant of a KOD DNA polymerase GH78 as set forth in SEQ ID NO: 1, wherein the first nucleotide sequence has amino-acid-codon substitution on a second nucleotide sequence encoding the KOD DNA polymerase GH78 at at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47 or 48 amino acid residues selected from a group consisting of positions 267, 326, 347, 349, 353, 375, 378, 379, 380, 385, 451, 452, 453, 454, 457, 461, 465, 470, 474, 477, 478, 479, 480, 482, 484, 485, 486, 493, 496, 497, 514, 574, 584, 605, 610, 630, 665, 666, 667, 674, 676, 680, 682, 698, 707, 718, 723 and 729,
the second nucleotide sequence is set forth in SEQ ID NO: 2,
optionally, the DNA molecule is further added with a second tag sequence at the terminal of the DNA molecule.

8. An expression cassette, a recombinant vector, a recombinant bacterium or a transgenic cell line comprising a DNA molecule of claim 6 or 7.

9. Use of a protein of any one of claims 1 to 4 in one or more of the following i-v:
i. serving as a DNA polymerase;
ii. catalyzing a DNA replication and/or DNA amplification;
iii. performing a nucleic acid sequencing;
iv. preparing a product for catalyzing a DNA replication and/or DNA amplification; and
v. preparing a product for a nucleic acid sequencing.

10. Use of a DNA molecule of claim 6 or 7 or an expression cassette, a recombinant vector, a recombinant bacterium or a transgenic cell line of claim 8 in one or more of the following i-vi:
i. preparing a DNA polymerase;
ii. catalyzing a DNA replication and/or DNA amplification;
iii. performing a nucleic acid sequencing;
iv. preparing a product of a DNA polymerase;
v. preparing a product for catalyzing a DNA replication and/or DNA amplification; and
vi. preparing a product for a nucleic acid sequencing.

11. The use of claim 9 or 10, wherein the sequencing is SBS sequencing, and the nucleic acid is a DNA, RNA or whole genome.
